(19) Europäisches Patentamt
European Patent Office
Office européen des brevets

(11) EP 2 912 015 B1

(12) **FASCICULE DE BREVET EUROPEEN**

(45) Date de publication et mention de la délivrance du brevet:
**21.02.2018 Bulletin 2018/08**

(21) Numéro de dépôt: **13783036.0**

(22) Date de dépôt: **23.10.2013**

(51) Int Cl.:
*C07C 323/58* (2006.01) *A61K 31/10* (2006.01)
*A61P 25/00* (2006.01) *A61P 27/00* (2006.01)

(86) Numéro de dépôt international:
**PCT/EP2013/072203**

(87) Numéro de publication internationale:
**WO 2014/064166 (01.05.2014 Gazette 2014/18)**

(54) **INHIBITEURS MIXTES DE L'AMINOPEPTIDASE N ET DE LA NÉPRILYSINE**

GEMISCHTE HEMMER DER AMINOPEPTIDASE N UND VON NEPRYLISIN

MIXED INHIBITORS OF AMINOPEPTIDASE N AND NEPRYLISIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.10.2012 FR 1260097**

(43) Date de publication de la demande:
**02.09.2015 Bulletin 2015/36**

(73) Titulaire: **Pharmaleads**
**75013 Paris (FR)**

(72) Inventeurs:
- **ROQUES, Bernard Pierre**
  **F-75014 Paris (FR)**
- **FOURNIE-ZALUSKI, Marie-Claude**
  **F-75011 Paris (FR)**
- **PORAS, Hervé**
  **F-78870 Bailly (FR)**

(74) Mandataire: **Regimbeau**
**20, rue de Chazelles**
**75847 Paris Cedex 17 (FR)**

(56) Documents cités:
**WO-A1-2009/138436 FR-A1- 2 892 413**

- **BERNARD P. ROQUES ET AL: "Inhibiting the breakdown of endogenous opioids and cannabinoids to alleviate pain", NATURE REVIEWS DRUG DISCOVERY, vol. 11, no. 4, 1 avril 2012 (2012-04-01), pages 292-310, XP055071274, ISSN: 1474-1776, DOI: 10.1038/nrd3673**
- **NOBLE FLORENCE ET AL: "Pain-suppressive effects on various nociceptive stimuli (thermal, chemical, electrical and inflammatory) of the first orally active enkephalin-metabolizing enzyme inhibitor RB 120", PAIN, ELSEVIER SCIENCE PUBLISHERS, AMSTERDAM, NL, vol. 73, no. 3, 1 janvier 1997 (1997-01-01), pages 383-391, XP002386725, ISSN: 0304-3959, DOI: 10.1016/S0304-3959(97)00125-5**
- **FOURNIE-ZALUSKI M-C ET AL: "MIXED INHIBITOR-PRODRUG AS A NEW APPROACH TOWARD SYSTEMICALLY ACTIVE INHIBITORS OF ENKEPHALIN-DEGRADING ENZYMES", JOURNAL OF MEDICINAL CHEMISTRY, AMERICAN CHEMICAL SOCIETY, US, vol. 35, no. 13, 1 janvier 1992 (1992-01-01), pages 2473-2481, XP002019768, ISSN: 0022-2623, DOI: 10.1021/JM00091A016**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 912 015 B1

- NOBLE F ET AL: "INHIBITION OF THE ENKEPHALIN-METABOLIZING ENZYMES BY THE FIRST SYSTEMICALLY ACTIVE MIXED INHIBITOR PRODRUG RB 101-INDUCES POTENT ANALGESIC RESPONSES IN MICE AND RATS", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 261, no. 1, 1 janvier 1992 (1992-01-01), pages 181-190, XP000386321, ISSN: 0022-3565

## Description

### DOMAINE DE L'INVENTION

L'invention concerne des inhibiteurs mixtes de l'aminopeptidase N et de la néprilysine, enzymes impliquées dans la dégradation des enképhalines.

### ARRIERE PLAN DE L'INVENTION

Les enképhalines, Tyr-Gly-Gly-Phe-Met(Leu) sont les ligands endogènes des récepteurs opioïdes $\mu$ et $\delta$ et sont impliquées dans la régulation des influx nociceptifs au niveau du système nerveux central comme au niveau périphérique. Cependant, administrés par voie i.c.v. à des rongeurs, ces peptides n'induisent qu'une réponse analgésique très brève, due à leur très rapide inactivation *in vivo,* y compris chez l'homme (Mosnaim et al. (2008) Neurochem. Res., 33, 81-86*)* bien que leur affinité pour les récepteurs opioïdes soit similaire à celle de la morphine. Deux métallopeptidases sont responsables de cette inactivation : l'aminopeptidase N (APN, EC 3.4.11.2) et la néprilysine (NEP, EC 3.4.24.11) qui clivent respectivement la liaison $Tyr^1$-$Gly^2$ et la liaison $Gly^3$-$Phe^4$ des enképhalines, conduisant ainsi à des métabolites inactifs (Roques et al. (1993) Pharmacol. Rev., 45, 87-146*).*

On connaît des inhibiteurs mixtes de ces deux enzymes, qui, en protégeant complètement les enképhalines endogènes de leur dégradation enzymatique, révèlent les activités pharmacologiques, en particulier analgésiques et antidépressives, des enképhalines (Noble et al. (2007) Expert. Opin. Ther. Targets, 11, 145-149*).* Ces inhibiteurs, décrits dans l'art antérieur, sont des hydroxamates (FR2518088 et FR2605004), des composés aminophosphiniques (FR2755135, FR2777780, FR0855015), des dérivés d'aminoacides à fonction thiol (FR2651229, FR0510862, FR0604030, FR0853092), des peptides endogènes (Wisner et al, PNAS (2006), 103, 17979-17984*).* Ces différentes molécules présentent des propriétés physicochimiques (solubilité) et pharmacodynamiques (biodisponibilité) qui leur octroient une efficacité pharmacologique par voie intraveineuse ou par voie orale sur différents type de douleurs, en particulier les douleurs aigues ou chroniques par excès de nociception (Noble et al. (2007) Expert. Opin. Ther. Targets, 11, 145-149) et neuropathiques (Menendez et al. (2008) Eur J Pharmacol, 596, 50-55 ; Thibault et al. (2008) Eur. J. Pharmacol., 600, 71-77*).*

Cependant, aucun des inhibiteurs mixtes décrits à ce jour, ne permet d'obtenir une réponse analgésique rapide, intense et avec une durée d'action assez longue dans le cas de douleurs vives (post-opératoires, cancéreuses, traumatiques, dentaires, etc.) après administration par voie intraveineuse, à des doses faibles permettant un usage en perfusion prolongée dans un véhicule utilisable en clinique.

L'objectif de l'invention est de fournir des composés présentant les propriétés bénéfiques des substances morphiniques sur le système nerveux central, en particulier l'analgésie, les effets comportementaux (diminution de la composante émotionnelle de la douleur et réponses antidépressives) sans leurs inconvénients majeurs centraux (accoutumance, dépendance physique et psychique, dépression respiratoire) et périphériques (constipation). D'autre part, il serait avantageux que les composés présentent des effets périphériques bénéfiques (antiinflammatoire et antineuropathique) sans les inconvenients énoncés ci-dessus.

### BREVE DESCRIPTION DE L'INVENTION

L'invention porte sur des composés possèdant la formule générale (I) suivante:

**(1)** $\mathbf{R}\text{-NH-CH}(\mathbf{R_1})\text{-CH}_2\text{-S-S-C}(\mathbf{R_2})(\mathbf{R_3})\text{-COCH}_2\text{-CH}(\mathbf{R_4})\text{-CO}\mathbf{R_5}$

avec $\mathbf{R}$, $\mathbf{R_1}$, $\mathbf{R_2}$, $\mathbf{R_3}$, $\mathbf{R_4}$ et $\mathbf{R_5}$ tels que définis dans la revendication 1.

L'invention porte également sur des compositions pharmaceutiques comprenant au moins un composé de la présente invention.

L'invention porte également sur des compositions pharmaceutiques comprenant au moins un composé de la présente invention et au moins un composé choisi parmi la morphine et ses dérivés, les endocannabinoïdes et les inhibiteurs de leur métabolisme, les dérivés du GABA tels que la gabapentine ou la prégabaline, la duloxetine ou la méthadone.

Enfin, l'invention porte sur les composés de la présente invention ou compositions pharmaceutiques les renfermant pour leur utilisation en tant que analgésique, anxiolytique, antidépresseur ou antiinflammatoire.

### BREVE DESCRIPTION DES FIGURES

Fig.1 : réponse antinociceptive induite après injection intraveineuse de composés selon la présente invention

(10 mg/kg) - Test de la plaque chaude chez la souris.

## DEFINITION

Les groupements alkyles désignent les chaînes hydrocarbonées linéaires ou ramifiées en C1, C2, C3, C4, C5 ou C6, en particulier les radicaux méthyle, éthyle, *n*-propyle, *i*-propyle, *n*-butyle, *i*-butyle ou *t*-butyle.

Des exemples d'hétérocycles à 5 ou 6 atomes, aromatiques ou saturés, comprenant au moins un atome de soufre, d'oxygène ou d'azote incluent les radicaux suivants : thiényle, pyrrolyle, imidazoyle, pyrazolyle, isothiazolyle, pyridyle, pyrazinyle, pyrimidinyle, pyridazinyle, pyrrolidinyle, pyrrolinyle, imidazolidinyle, pyrazolidinyle, pyrazolinyle, piperidyle, piperazinyle, thiadiazolyle, furyle, pyranyle, isoxazolyle, morpholinyle, furazanyle, oxazolyle, oxazolidinyle et oxazolinyle.

Le terme « halogène » tel que désigné ici désigne un chlore, un brome, un iode ou un fluor.

## DESCRIPTION DETAILLEE DE L'INVENTION

Les composés de la présente invention correspondent à l'association, par l'intermédiaire d'un pont disulfure, d'un inhibiteur d'APN et d'un inhibiteur de NEP, capables d'inhiber l'activité de ces deux enzymes à des concentrations nanomolaires. Ce pont disulfure est clivé *in vivo* et libère les deux inhibiteurs qui interagiront avec leur cible respective (NEP ou APN) (Fournié-Zaluski et al. (1992) J. Med. Chem., 35, 2473-2481*).*

Les inhibiteurs de NEP décrits à ce jour possèdent généralement un motif peptidique et donc une ou plusieurs liaison(s) amide(s). Par exemple, des dérivés peptidomimétiques comprenant au moins une liaison amide et comprenant en outre un motif disulfide ont été décrits dans les articles Roques et al (Nature Rev. Drug Discov. (2012) 11, 292-311), Noble et al 1992 (Journal of Pharmacology and Experimental Therapeutics 261 (1992), 1, 181-190), Noble et al 1997 (Pain 73 (97), 383-391) ou , et dans les documents brevet WO 2009/138436 et FR 2892413. De plus ils possèdent généralement un poids moléculaire (PM) supérieur à 500 Da. Ces caractéristiques sont peu favorables au franchissement des barrières physiologiques, intestinale par exemple et ces produits présentent de ce fait une biodisponibilité orale assez faible. Il en est de même pour le franchissement de la barrière hémato-méningée.

La structure des inhibiteurs de NEP utiles dans la présente invention et permettant de pallier ces inconvénients se caractérise par: i) un motif thio-cétonique original capable d'interagir avec le zinc de la NEP de manière mono ou bidentée, ii) une absence de motif peptidique (donc dépourvu de liaison amide), iii) un squelette comportant un minimum de groupes permettant d'atteindre une affinité nanomolaire pour la NEP, iv) un faible poids moléculaire.

Les composés de la présente invention possèdent la formule générale **(1)** suivante:

**(1)** $\mathbf{R}\text{-NH-CH}(\mathbf{R_1})\text{-CH}_2\text{-S-S-C}(\mathbf{R_2})(\mathbf{R_3})\text{-COCH}_2\text{-CH}(\mathbf{R_4})\text{-CO}\mathbf{R_5}$

dans laquelle :

a) **R** représente :

- un hydrogène ;
- un groupement alkoxyalkylcarbonyl R'C(O)OCH(R")OC(O)- où R' et R" représentent, indépendamment l'un de l'autre, un groupement alkyle contenant de 1 à 6 atomes de carbone ;

b) $\mathbf{R_1}$ représente un groupement alkyle, linéaire ou ramifié, de 1 à 6 atomes de carbone, substitué ou non, par un groupement -OR'", -SOR'" ou -SR'", avec R'" représentant un groupement alkyle de 1 à 6 atomes de carbone, substitué ou non, par un ou plusieurs atomes d'halogènes ;

c) $\mathbf{R_2}$ représente :

- un groupement alkyle, linéaire ou ramifié, de 1 à 6 atomes de carbone, substitué ou non, par:
  - un groupement $-OR_6$, $-SR_6$ ou $-SOR_6$, avec $R_6$ représentant un hydrogène, un groupement alkyle linéaire ou ramifié de 1 à 4 carbones, un groupement phényle ou benzyle ;
  - un groupement $-CO_2R_7$, avec $R_7$ représentant un hydrogène, un groupement alkyle, linéaire ou ramifié, comprenant de 2 à 4 atomes de carbone ou un groupement benzyle ;
  - un groupement $-NR_8R_9$, avec $R_8$ et $R_9$ représentant indépendamment l'un de l'autre un hydrogène, un groupement alkyle, linéaire ou ramifié, de 1 à 4 atomes de carbone, un groupement phényle ou benzyle, ou avec $-NR_8R_9$ pris ensemble représentant un hétérocycle saturé à 5 ou 6 chaînons comportant un ou plusieurs hétéroatomes choisis parmi N ou O, de préférence représentant une morpholine ou une pipéridine;

ou

- un groupement carboxamide $-CONR_8R_9$, avec $-NR_8R_9$ tel que défini ci-dessus ;
- un groupement phényle, substitué, ou non, par un ou plusieurs halogènes pris parmi le fluor ou le brome, un groupement alkoxy $-OR_6$, avec $R_6$ ayant la même définition que ci-dessus ou par un groupement phényle;
- un hétérocycle aromatique à 5 ou 6 chaînons comprenant 1 ou 2 hétéroatomes choisi(s) parmi l'oxygène, l'azote et le soufre;
- un composé cyclique saturé à 5 ou 6 chaînons ou hétérocycle saturé à 5 ou 6 chaînons comprenant 1 ou 2 hétéréatomes choisis parmi l'oxygène, l'azote et le soufre ;

- un composé cyclique saturé à 5 ou 6 chaînons ou hétérocyclique saturé à 5 ou 6 chaînons comprenant 1 ou 2 hétéroatomes choisis parmi l'oxygène, l'azote et le soufre ; ou
- un groupement phényle substitué ou non par un ou plusieurs halogènes pris parmi le fluor ou le brome, ou par un groupement $-OR_5$ avec $R_5$ ayant la même définition que ci-dessus ;

et $\mathbf{R_3}$ représente un hydrogène ; ou $\mathbf{R_2}$ et $\mathbf{R_3}$ sont identiques et représentent un groupement alkyle, linéaire ou ramifié de 1 à 6 atomes de carbone ; ou

$\mathbf{-C(R_2)(R_3)-}$ pris ensemble représente :

- un composé cyclique saturé à 5 chaînons accolé, ou non, à un cycle aromatique (conduisant par exemple au cycle indanyle);
- un composé cyclique saturé à 6 chaînons ;
- un composé hétérocyclique saturé à 6 chaînons comprenant 1 hétéroatome, en position 4, choisi parmi l'oxygène, l'azote et le soufre, dans lequel lorsque l'hétéroatome est l'azote, l'azote est substitué, ou non, par un groupement alkyle de 1 à 6 atomes de carbone, un groupement phényle, benzyle ou un groupement alcanoyle;

d) $\mathbf{R_4}$ représente :

- un groupement alkyle, linéaire ou ramifié, de 1 à 6 atomes de carbone, substitué ou non, par:

  - un groupement $-OR_6$, $-SR_6$ ou $-SOR_6$, avec $R_6$ représentant un hydrogène, un groupement alkyle linéaire ou ramifié de 1 à 4 carbones, un groupement phényle ou benzyle ;
  - un groupement $-CO_2R_7$, avec $R_7$ représentant un hydrogène, un groupement alkyle, linéaire ou ramifié, comprenant de 2 à 4 atomes de carbone, un groupement benzyle ;
  - un groupement $-NR_8R_9$, avec $R_8$ et $R_9$ représentant indépendamment l'un de l'autre un hydrogène, un groupement alkyle, linéaire ou ramifié, de 1 à 4 atomes de carbone, un groupement phényle ou benzyle, ou avec $-NR_8R_9$ pris ensemble représentant un hétérocycle saturé à 5 ou 6 chaînons comportant un ou plusieurs hétéroatomes choisis parmi N ou O, de préférence représentant une morpholine ou une pipéridine;
  - un groupement carboxamide $-CONR_8R_9$, avec $-NR_8R_9$ tel que défini ci-dessus ;
  - un groupement phényle substitué, ou non, par :

    - un ou plusieurs halogènes pris parmi le fluor ou le brome ;
    - un groupement $-OR_6$, $R_5$ ayant la même définition que ci-dessus ;
    - un groupement phényle ou thiényle ;

  - un hétérocycle aromatique à 5 ou 6 chaînons comprenant 1 ou 2 hétéroatomes choisi(s) parmi l'oxygène, l'azote et le soufre ; ou
  - un composé cyclique saturé à 5 ou 6 chaînons ou hétérocyclique saturé à 5 ou 6 chaînons comprenant 1 ou 2 hétéroatomes choisis parmi l'oxygène, l'azote et le soufre ;

- un groupement phényle, substitué ou non, par :

  - un ou plusieurs halogènes notamment le fluor ou le brome ;
  - un groupement $-OR_6$, avec $R_6$ ayant la même définition que ci-dessus ;
  - un phényle ;
  - un hétérocycle aromatique à 5 ou 6 chaînons comprenant 1 ou 2 hétéroatomes choisis parmi l'oxygène, l'azote et le soufre ;

e) $\mathbf{R_5}$ représente :

- un groupement hydroxyle ;
- un groupement $-NR_8R_9$, avec $R_8$ et $R_9$ représentant indépendamment l'un de l'autre un hydrogène, un groupement alkyle, linéaire ou ramifié, de 1 à 4 atomes de carbone, un groupement phényle ou benzyle, ou avec $-NR_8R_9$ pris ensemble représentant un hétérocycle à 5 ou 6 chaînons, comportant un ou plusieurs hétéroatomes choisis parmi N ou O, de préférence représentant une morpholine ou une pipéridine ;
- un groupement alkoxy $-OR_{10}$, avec $R_{10}$ représentant :

  - un groupement alkyle, linéaire ou ramifié, comportant de 2 à 6 atomes de carbone ;
  - un groupement benzyle ;
  - un groupement $-CHR_{11}-COOR_{12}$, $-CHR_{11}-O-C(=O)R_{12}$, $-CHR_{11}$-ou $-C(=O)-OR_{12}$ dans lesquels $R_{11}$ et $R_{12}$ représentent, indépendamment l'un de l'autre, un groupement alkyle, linéaire ou ramifié, de 1 à 6 atomes de carbone.

Les composés de la présente invention peuvent être sous forme de sels d'addition pharmacologiquement acceptables, tels que les sels d'addition des composés de formule **(1)** avec des acides minéraux ou organiques lorsque la fonction amine est libre ou des bases minérales ou organiques lorsque la fonction acide est libre.

Les protections des parties N-terminale et/ou C-terminale par les groupements R et $R_5$ sont généralement réalisées pour favoriser la biodisponibilité par les différentes voies d'administration.

Dans des réalisations particulières de l'invention, les composés possèdent la formule générale **(1)** dans laquelle $\mathbf{R_2}$ représente un groupement alkyle linéaire ou ramifié de 1 à 6 atomes de carbone ou un groupement alkyle linéaire ou ramifié de 1 à 6 atomes de carbone substitué par:

- un groupement phényle ;
- un groupement phényle substitué par un ou plusieurs halogènes pris parmi le fluor ou le brome ;
- un hétérocycle aromatique à 5 ou 6 chaînons comprenant 1 ou 2 hétéroatomes choisi(s) parmi l'oxygène, l'azote et le soufre ;
- un composé cyclique saturé à 5 ou 6 chaînons ou hétérocyclique saturé à 5 ou 6 chaînons comprenant 1 ou 2 hétéroatomes choisis parmi l'oxygène, l'azote et le soufre ;

et $\mathbf{R_3}$ représente un hydrogène ; ou
$\mathbf{R_2}$ et $\mathbf{R_3}$ sont identiques et représentent un groupement alkyle linéaire ou ramifié de 1 à 6 atomes de carbone, ou **$-C(R_2)(R_3)-$** pris ensemble représente :

- un composé cyclique saturé à 5 chaînons,
- un composé cyclique saturé à 5 chaînons accolé à un cycle aromatique ;
- un composé cyclique saturé à 6 chaînons ; ou
- un composé hétérocyclique saturé à 6 chaînons comprenant 1 hétéroatome, en position 4, choisi parmi l'oxygène, l'azote et le soufre ;

et **R, $\mathbf{R_1}$, $\mathbf{R_4}$, $\mathbf{R_5}$** sont tels que décrits ci-dessus ou ci-après.

Plus particulièrement, $\mathbf{R_2}$ peut représenter un groupement isobutyle ou un groupement méthyle substitué par:

- un groupement phényle;
- un groupement phényle substitué en position 4 par un halogène pris parmi le fluor ou le brome;
- un groupement phényle substitué en position 4 par un groupement phényle;

et $\mathbf{R_3}$ représente un hydrogène, ou
$\mathbf{R_2}$ et $\mathbf{R_3}$ sont identiques et représentent un groupement méthyle ou éthyle, ou
**$-C(R_2)(R_3)-$** représentent ensemble:

- un groupement cyclique saturé à 5 ou 6 chaînons; ou
- un groupement cyclique saturé à 5 chaînons accolé à un cycle aromatique.

Dans des réalisations particulières de l'invention,, les composés possèdent la formule générale **(1)** dans laquelle $\mathbf{R_4}$ représente un groupement alkyle linéaire ou ramifié de 1 à 6 atomes de carbones substitué par:

- un groupement phényle, non substitué ou substitué par un ou plusieurs halogènes pris parmi le fluor ou le brome, un groupement alkoxy $-OR_6$, avec $R_6$ ayant la même définition que ci-dessus, ou par un groupement phényle;

- un hétérocycle aromatique à 5 ou 6 chaînons comprenant 1 ou 2 hétéroatomes choisi(s) parmi l'oxygène, l'azote et le soufre;
- un composé cyclique saturé à 5 ou 6 chaînons ;
- un composé cyclique hétérocycle saturé à 5 ou 6 chaînons comprenant 1 ou 2 hétéréatomes choisi(s) parmi l'oxygène, l'azote et le soufre ;

et **R, $R_1$ $R_2$, $R_3$, $R_5$** sont tels que décrits ci-dessus ou ci-après.

Avantageusement, **$R_4$** représente un groupement alkyle linéaire ou ramifié de 1 à 6 atomes de carbones substitué par:

- un groupement phényle; ou
- un groupement phényle substitué par:
  - un ou plusieurs halogènes pris parmi le fluor ou le brome;
  - un groupement phényle ou thiényle,

et **R, $R_1$, $R_2$, $R_3$, $R_5$** sont tels que décrits ci-dessus ou ci-après.

Plus particulièrement, **$R_4$** peut représenter un groupement alkyle à un carbone substitué par:

- un groupement phényle;
- un groupement phényle substitué en position 4 par un halogène pris parmi le fluor ou le brome;
- un groupement phényle substitué en position 4 par un groupement phényle.

Dans des réalisations particulières de l'invention, les composés possèdent la formule générale **(1)** dans laquelle **$R_5$** représente :

- un groupement hydroxyle ; ou
- un groupement alkoxy $-OR_{10}$, avec $R_{10}$ représentant :
  - un groupement alkyle, linéaire ou ramifié, comportant de 2 à 6 atomes de carbone ;
  - un groupement benzyle ;

un groupement $-CHR_{11}-COOR_{12}$, $-CHR_{11}-O-C(=O)R_{12}$, $-CHR_{11}$-ou $-C(=O)-OR_{12}$ dans lesquels $R_{11}$ et $R_{12}$ représentent, indépendamment l'un de l'autre, un groupement alkyle, linéaire ou ramifié, de 1 à 6 atomes de carbone ;
et **R, $R_1$, $R_2$, $R_3$, $R_4$** sont tels que décrits ci-dessus ou ci-après

Avantageusement, **$R_5$** représente un groupement hydroxyle et **R, $R_1$, $R_2$, $R_3$, $R_4$** sont tels que décrits ci-dessus ou ci-après.

Des composés préférés possèdent la formule générale (1) dans laquelle

a) **$R_1$** est choisi parmi $-CH_2CH_2SCH_3$, $-CH_2CH_2CH_2CH_3$, $-CH_2CH_2SOCH_3$;
b) **$R_2$** représente un groupement alkyle linéaire ou ramifié de 1 à 6 atomes de carbone ou un groupement alkyle linéaire ou ramifié de 1 à 6 atomes de carbone substitué par:

- un groupement phényle ;
- un groupement phényle substitué par un ou plusieurs halogènes pris parmi le fluor ou le brome ;
- un hétérocycle aromatique à 5 ou 6 chaînons comprenant 1 ou 2 hétéroatomes choisi(s) parmi l'oxygène, l'azote et le soufre ;
- un composé cyclique saturé à 5 ou 6 chaînons ou hétérocyclique saturé à 5 ou 6 chaînons comprenant 1 ou 2 hétéroatomes choisis parmi l'oxygène, l'azote et le soufre ;

et **$R_3$** représente un hydrogène ; ou
**$R_2$** et **$R_3$** sont identiques et représentent un groupement alkyle linéaire ou ramifié de 1 à 6 atomes de carbone ; ou
**$-C(R_2)(R_3)-$** pris ensemble représente :

- un composé cyclique saturé à 5 chaînons,
- un composé cyclique saturé à 5 chaînons accolé à un cycle aromatique ;
- un composé cyclique saturé à 6 chaînons ; ou
- un composé hétérocyclique saturé à 6 chaînons comprenant 1 hétéroatome, en position 4, choisi parmi

l'oxygène, l'azote et le soufre ;

c) $\mathbf{R_4}$ représente un groupement alkyle linéaire ou ramifié de 1 à 6 atomes de carbone substitué par:

- un groupement phényle ; ou
- un groupement phényle substitué par :

  - un ou plusieurs halogènes pris parmi le fluor ou le brome ;
  - un groupement phényle ou thiényle ;

d) $\mathbf{R_5}$ représente un groupement hydroxyle.

Des composés préférés possèdent la formule **(1)** dans laquelle :

- $\mathbf{R_2}$ = $CH_2Ph$; $\mathbf{R_3}$ = H; $\mathbf{R_4}$ = $CH_2Ph$ ; ou
- $\mathbf{R_2}$ = iBu; $\mathbf{R_3}$ = H; $\mathbf{R_4}$ = $CH_2$(4-Br-Ph); ou
- $\mathbf{R_2}$ = $CH_2$(4-Br-Ph); $\mathbf{R_3}$ = H; $\mathbf{R_4}$ = $CH_2$(4-Br-Ph); ou
- $\mathbf{R_2}$ = $CH_2$(4-Br-Ph); $\mathbf{R_3}$ = H; $\mathbf{R_4}$ = $CH_2Ph$; ou
- $\mathbf{R_2}$ = $CH_2Ph$; $\mathbf{R_3}$ = H; $\mathbf{R_4}$ = $CH_2$(4-Br-Ph); ou
- $\mathbf{R_2}$ = $CH_2$(4-Ph-Ph); $\mathbf{R_3}$ = H; $\mathbf{R_4}$ = $CH_2$(4-Br-Ph); ou
- $\mathbf{R_2}$ = $CH_3$; $\mathbf{R_3}$ = $CH_3$; $\mathbf{R_4}$ = $CH_2$(4-Br-Ph); ou
- $\mathbf{R_2}$ = $C_2H_5$; $\mathbf{R_3}$ = $C_2H_5$; $\mathbf{R_4}$ = $CH_2$(4-Br-Ph); ou
- $\mathbf{C(R_2)(R_3)}$ = cyclopentyle; $\mathbf{R_4}$ = $CH_2$(4-Br-Ph); ou
- $\mathbf{C(R_2)(R_3)}$ = cyclohexyle; $\mathbf{R_4}$ = $CH_2$(4-Br-Ph); ou
- $\mathbf{C(R_2)(R_3)}$ = indanyle; $\mathbf{R_4}$ = $CH_2$(4-Br-Ph); ou
- $\mathbf{C(R_2)(R_3)}$ = cyclohexyle; $\mathbf{R_4}$ = $CH_2$(4-Ph-Ph); ou
- $\mathbf{C(R_2)(R_3)}$ = indanyle; $\mathbf{R_4}$ = $CH_2$(4-Ph-Ph).

Dans des modes de réalisation particuliers, les composés de la présente invention possèdent la formule **(1)** dans laquelle $\mathbf{R_1}$ est choisi parmi $-CH_2CH_2SCH_3$, $-CH_2CH_2CH_2CH_3$,$-CH_2CH_2SOCH_3$.

Dans des modes de réalisation préférés, les composés de la présente invention possèdent la formule **(1)** dans laquelle $\mathbf{R_1}$ est choisi parmi $-CH_2CH_2SCH_3$, $-CH_2CH_2CH_2CH_3$,$-CH_2CH_2SOCH_3$, et

- $\mathbf{R_2}$ = $CH_2Ph$; $\mathbf{R_3}$ = H; $\mathbf{R_4}$ = $CH_2Ph$ ; ou
- $\mathbf{R_2}$ = iBu; $\mathbf{R_3}$ = H; $\mathbf{R_4}$ = $CH_2$(4-Br-Ph); ou
- $\mathbf{R_2}$ = $CH_2$(4-Br-Ph); $\mathbf{R_3}$ = H; $\mathbf{R_4}$ = $CH_2$(4-Br-Ph); ou
- $\mathbf{R_2}$ = $CH_2$(4-Br-Ph); $\mathbf{R_3}$ = H; $\mathbf{R_4}$ = $CH_2Ph$;
- $\mathbf{R_2}$ = $CH_2Ph$; $\mathbf{R_3}$ = H; $\mathbf{R_4}$ = $CH_2$(4-Br-Ph); ou
- $\mathbf{R_2}$ = $CH_2$(4-Ph-Ph); $\mathbf{R_3}$ = H; $\mathbf{R_4}$ = $CH_2$(4-Br-Ph); ou
- $\mathbf{R_2}$ = $CH_3$; $\mathbf{R_3}$ = $CH_3$; $\mathbf{R_4}$ = $CH_2$(4-Br-Ph); ou
- $\mathbf{R_2}$ = $C_2H_5$; $\mathbf{R_3}$ = $C_2H_5$; $\mathbf{R_4}$ = $CH_2$(4-Br-Ph); ou
- $\mathbf{C(R_2)(R_3)}$ -= cyclopentyle; $\mathbf{R_4}$ = $CH_2$(4-Br-Ph); ou
- $\mathbf{C(R_2)(R_3)}$ = cyclohexyle; $\mathbf{R_4}$ = $CH_2$(4-Br-Ph); ou
- $\mathbf{C(R_2)(R_3)}$ = indanyle; $\mathbf{R_4}$ = $CH_2$(4-Br-Ph); ou
- $\mathbf{C(R_2)(R_3)}$ = cyclohexyle; $\mathbf{R_4}$ = $CH_2$(4-Ph-Ph); ou
- $\mathbf{C(R_2)(R_3)}$ = indanyle; $\mathbf{R_4}$ = $CH_2$(4-Ph-Ph).

Dans d'autres modes de réalisation, les composés de la présente invention possèdent la formule **(1)** dans laquelle:

- $\mathbf{R_1}$ = $CH_2CH_2SCH_3$; $\mathbf{R_2}$ = $CH_2CH(CH_3)_2$; $\mathbf{R_3}$= H; $\mathbf{R_4}$ = $CH_2$(4-Br-Ph);
- $\mathbf{R_1}$ = $CH_2CH_2CH_2CH_3$; $\mathbf{R_2}$ = $CH_2CH(CH_3)_2$; $\mathbf{R_3}$= H; $\mathbf{R_4}$ = $CH_2$(4-Br-Ph);
- $\mathbf{R_1}$ = $CH_2CH_2SOCH_3$; $\mathbf{R_2}$ = $CH_2CH(CH_3)_2$; $\mathbf{R_3}$= H; $\mathbf{R_4}$ = $CH_2$(4-Br-Ph);
- $\mathbf{R_1}$ = $CH_2CH_2CH_2CH_3$; $\mathbf{-C(R_2R_3)-}$= Cyclohexyle; $\mathbf{R_4}$ = $CH_2$(4-Br-Ph);
- $\mathbf{R_1}$ = $CH_2CH_2SCH_3$; $\mathbf{-C(R_2)(R_3)-}$= Cyclohexyle; $\mathbf{R_4}$ = $CH_2$(4-Ph-Ph); ou
- $\mathbf{R_1}$ = $CH_2CH_2CH_2CH_3$; $\mathbf{-C(R_2)(R_3)-}$= Cyclohexyle; $\mathbf{R_4}$ = $CH_2$(4-Ph-Ph);

et **R** et $\mathbf{R_5}$ sont tels que décrits ci-dessus.

Dans des modes de réalisation particuliers de l'invention, les composés de la présente invention possèdent la formule **(1)** dans laquelle **R** est un hydrogène ou **R** est un groupement R'C(O)OCH(R")OC(O)- dans lequel R' est un groupement isopropyle et R" un groupement méthyle, et $\mathbf{R_1}$, $\mathbf{R_2}$, $\mathbf{R_3}$, $\mathbf{R_4}$ et $\mathbf{R_5}$ sont tels que décrits ci-dessus.

Les composés de la présente invention peuvent être utilisés en tant que médicament. Plus particulièrement, les composés peuvent être employés pour préparer des compositions pharmaceutiques comprenant à titre de principe actif au moins un des composés décrits ci-dessus en combinaison avec au moins un excipient pharmaceutiquement acceptable. Lesdits excipients sont choisis selon la forme pharmaceutique et le mode d'administration souhaité parmi les excipients habituels qui sont connus de l'homme du métier.

Les composés de la présente invention inhibant conjointement les activités enzymatiques responsables de la dégradation des enképhalines, ils augmentent leur taux endogène extracellulaire et s'avèrent à ce titre être des analgésiques et/ou antidépresseurs efficaces. Les effets analgésiques des composés se manifestent sur diverses douleurs, aiguës ou chroniques, telles que les douleurs neurogéniques, neuropathiques, neuroinflammatoires, nociceptives ou générale comme la fibromyalgie. Des exemples de douleur incluent les douleurs mécaniques (par exemple douleur musculaire, ischémie d'origine vasculaire), les douleurs des membres amputés, les douleurs occasionnées par un zona, les douleurs cancéreuses liées au cancer lui-même ou aux conséquences des traitements, les douleurs associées aux maladies inflammatoires (par exemple, arthrite, arthrite rhumatoïde, ostéoarthrite, goutte), les douleurs liées au diabète insulinique, les douleurs liés aux migraines, aux névralgie faciales, céphalées, les douleurs liées aux atteintes des nerfs périphériques (par exemple post-opératoire), les névralgies dorsales, les douleurs dentaires, les douleurs liées aux brûlures, coups de soleil, morsures ou piqûres, les douleurs liées aux infections, troubles métaboliques (diabète, alcoolisme), compression nerveuse (hernie, canal carpien, fibrose...), fractures, brûlures, hématomes, coupures et inflammation.

Enfin, typiquement, et de manière avantageuse, les composés de la présente invention ne présentent pas les inconvénients majeurs des substances morphiniques (tolérance, dépendance physique, dépression respiratoire, nausée, sédation, constipation, ...).

Ainsi, les composés de la présente invention et les compositions pharmaceutiques les renfermant peuvent utiles pour au moins une utilisation choisie parmi les utilisations suivantes : analgésique, anxiolytique, antidépresseur ou antiinflammatoire.

La présente invention concerne également l'utilisation des composés de formule (I) telle que définie ci-dessus et les compositions pharmaceutiques les renfermant pour la fabrication d'un médicament analgésique, anxiolytique, antidépresseur ou anti-inflammatoire, plus particulièrement d'un médicament destiné au traitement de la douleur. La douleur peut être notamment une douleur chronique ou aiguë telle que définie ci-dessus.

Les composés de la présente invention peuvent être utilisés seuls ou en combinaison avec des composés connus pour leurs propriétés antinociceptives. Cette combinaison peut permettre une potentialisation des effets pharmacologiques, d'autant que les composés antinociceptifs connus présentent généralement à fortes doses des effets secondaires indésirables.

De telles potentialisations (synergies) des effets pharmacologiques ont été démontrées dans le passé en combinant des inhibiteurs mixtes présentant une structure chimique différente de celle des inhibiteurs mixtes de la présente invention avec des composés antinociceptifs connus. Ainsi, une forte potentialisation des réponses antinociceptives a été obtenue, par exemple, par combinaison avec: la morphine (Mas Nieto et al. (2001) Neuropharmacol. 41, 496-506, le THC (Valverde et al. (2001) Eur. J. Neurosci., 13, 1816-1824), la gabapentine (Menendez et al. (2007) Eur. J. Pharmacol., 596, 50-55) et ses analogues tel que la prégabaline. Ces associations permettent pour un effet pharmacologique équivalent de réduire de 3 à 10 fois les doses des composants de l'association (morphine et inhibiteur par exemple). Ainsi, dans un mode de réalisation, les compositions pharmaceutiques comprennent à titre de principe actif au moins un des composés de la présente invention en combinaison avec au moins un antinociceptif et au moins un excipient pharmaceutiquement acceptable. Les antinociceptifs peuvent être choisis parmi :

- la morphine et ses dérivés,
- les endocannabinoïdes, le $\Delta^9$ THC, les agonistes des récepteurs cannabinoïdes synthétiques ou les inhibiteurs de la dégradation de l'anandamide (FAAH), ou
- les analogues du GABA, tels que la gabapentine ou la prégabaline, ou
- la duloxetine, inhibiteur de la recapture de la sérotonine et de la noradrénaline.

Dans un autre mode de réalisation, les compositions pharmaceutiques comprennent à titre de principe actif au moins un des composés de la présente invention en combinaison avec la méthadone et au moins un excipient pharmaceutiquement acceptable.

Dans un autre mode de réalisation, la présente invention concerne une composition comprenant:

a) au moins un composé de formule (1) telle que définie ci-dessus, et
b) au moins un antinociceptif, par exemple choisi parmi la morphine et ses dérivés, les endocannabinoïdes, le $\Delta^9$

THC, les agonistes des récepteurs cannabinoïdes synthétiques ou les inhibiteurs de la dégradation de l'anandamide (FAAH), ou analogues du GABA, tels que la gabapentine ou la prégabaline, ou la duloxetine,

en tant que produit de combinaison pour une utilisation simultanée, spéarée ou étalée dans le temps pour le traitement de la douleur, en particulier des douleurs chroniques ou aiguës.

Dans le passé, il a été montré que l'association d'un inhibiteur mixte présentant une structure chimique différente de celle des composés de la présente invention avec la méthadone permettait d'amplifier de manière synergique l'action des constituants (Le Guen et al. (2003) Pain, 104, 139-148). Cette association réduit les processus addictifs aux opiacés et à la cocaïne.

Les compositions pharmaceutiques selon l'invention peuvent être administrées par voie parentérale, telle que par voie intraveineuse ou intradermique, ou par voie topique, orale ou nasale.

Les formes administrables par voie parentérale incluent les suspensions aqueuses, les solutions salines isotoniques ou les solutions stériles et injectables qui peuvent contenir des agents de dispersion et/ou des mouillants pharmacologiquement compatibles. Les formes administrables par voie orale incluent les comprimés, les gélules molles ou dures, les poudres, les granules, les solutions et suspensions orales. Les formes administrables par voie nasale incluent les aérosols. Les formes administrables par voie topique incluent les patchs, les gels, les crèmes, les pommades, les lotions, les sprays, les collyres.

La dose efficace d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter.

La présente invention, selon un autre de ses aspects, concerne également une méthode de traitement des pathologies ci-dessus indiquées qui comprend l'administration, à un patient en ayant besoin, d'une dose efficace d'un composé selon l'invention, ou un de ses sels pharmacetiquement acceptable ou d'une composition selon l'invention, de préférence par voie parentérale, voie orale ou nasale.

Les inhibiteurs mixtes NEP-APN **1** peuvent être préparés en deux étapes. Dans une première étape, le Boc béta-aminothiol **11** (Fournié-Zaluski M-C. et al (1992) J. Med. Chem., 35, 2473-2481*)* est activé au moyen du chlorure de l'acide methoxycarbonylsulfonique puis dans une deuxième étape, est condensé avec les acides mercaptoalcanoïques **10** pour donner les composés **12.**

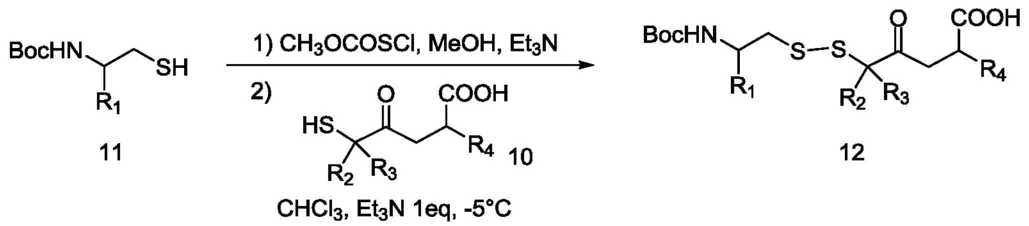


11 10 12

Les esters **13** sont obtenus à partir des acides **12** par réaction avec l'alcool $R_5OH$ correspondant ou par reflux dans l'acétate d'éthyle avec le dérivé chloré $R_5Cl$ en présence de $Et_3N$.

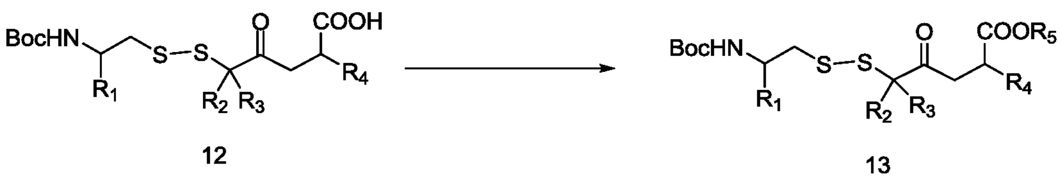

12 13

La déprotection du groupement N-terminal Boc de **13** est réalisée par action de l'acide formique, libérant **1.**

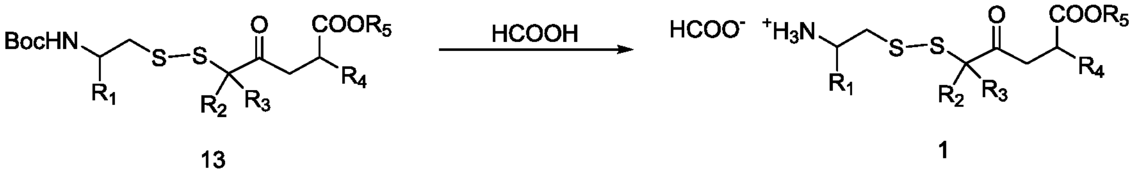


13 1

Alternativement, les esters **1** peuvent être obtenus à partir de **12,** par déprotection du groupement N-terminal

Boc par action de l'acide formique suivi par une estérification par l'alcool correspondant en présence de $SOCl_2$ à température ambiante.

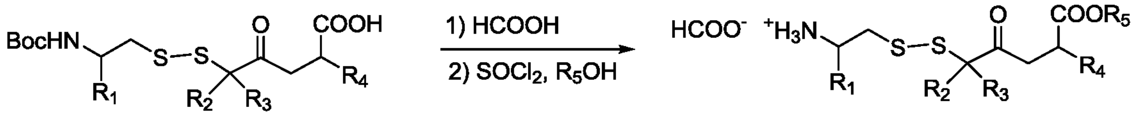


12 1

Alternativement, le composé N-protégé **1** (R = iPrCOOCH($CH_3$)OCO) peut être obtenu à partir de **12,** par déprotection du groupement N-terminal Boc par action de l'acide formique suivi une condensation avec 1-((2, 5-dioxopyrrolidin-1-yloxy)carbonyloxy)ethyl isobutyrate dans $CH_3CN$ en présence de $NaHCO_3$ 2N (Cundy et al. (2004) J. Pharm. Exp. Therap., 311, 315-323).

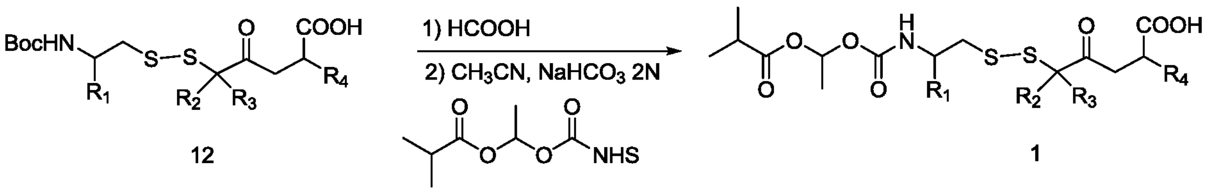


12 1

Les composés de formule **10** pour lesquels $R_3$ = H et $R_5$ = OH peuvent être obtenus en 5 étapes à partir d'un acide aminé **2** de configuration absolue définie, de préférence (*R*).

**Etape 1:**

L'acide aminé **2** est transformé en dérivé bromé **3** par réaction de déamination-halogénation, qui, généralement, se fait avec rétention de configuration (Claeson G. et al. (1968) Acta Chem. Scand. 22, 3155-3159; Dutta A. et al. (1987) J. Chem. Soc. Perkin Trans. 1, 111-120).

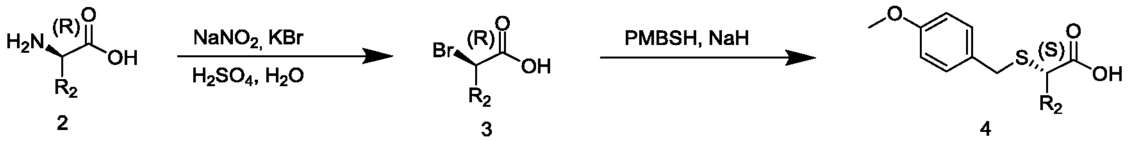


2 3 4

Le dérivé bromé **3** est transformé en thioether **4** par substitution nucléophile avec inversion de configuration par action du 4-methoxy-$\alpha$-toluenethiol (PMBSH) en milieu basique.

**Etape 2: Préparation de l'halogénométhyl cétone 6 à partir de 4**

Procédé 1: le composé **4** est transformé en cétène **5** soit à partir de l'anhydride mixte de **4** (préparé par action de l'isobutylchloroformiate et de N-méthylmorpholine), soit à partir du chlorure d'acide (préparé par action du chlorure de thionyle sur **4**).

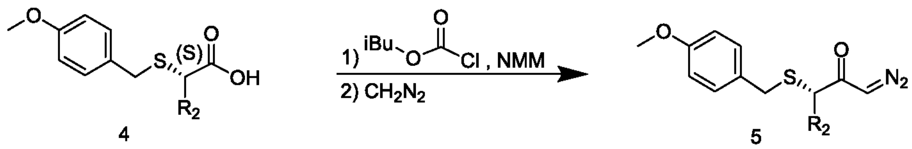


4 5

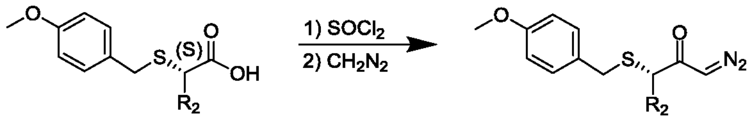


4 5

Le cétène **5** est ensuite transformé en halogenométhylcétone **6** par bullage d'HCl gaz ou d'HBr gaz dans le 1,4-Dioxanne, le diethyléther ou l'acétate d'éthyle.

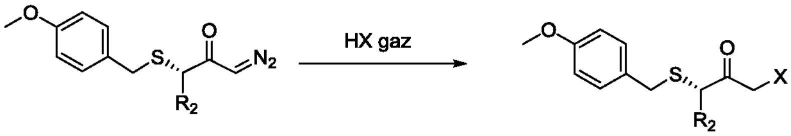


5 6

Procédé 2: alternativement la chlorométhylcétone **6** (X=Cl) peut être obtenue par action du chloroiodométhane sur l'ester méthylique de **4** (préparé en présence de DMAP et d'EDCI ou bien encore par action du chlorure d'acétyle dans le méthanol) en présence de LDA fraîchement préparé (Chen et al. (1997) Tet. Lett. 38, 18, 3175-3178).

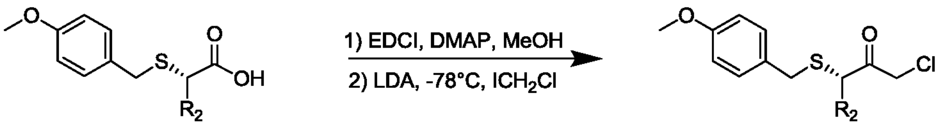


4 6

## Etape 3

Procédé 1: l'halogénométhylcétone **6,** traitée par NaI, subit un échange d'halogène et réagit ensuite sur le sel de sodium du dialkylmalonate pour donner le composé **7.** $R_{13}$ peut être un groupement Méthyle, Ethyle ou tert-Butyle.

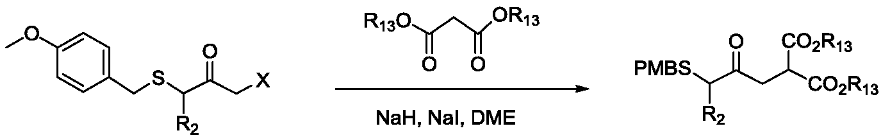


6 7

Le substituant $R_4$ est introduit par action d'un dérivé bromé $R_4Br$ sur l'anion du malonate précédent **7,** déprotoné *in situ* par NaH. On obtient le composé **8.**

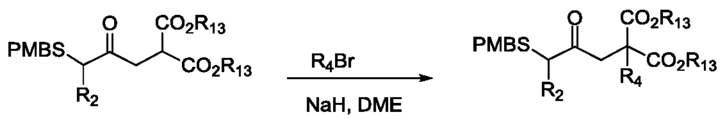


7 8

Procédé 2: le substituant $R_4$ peut également être introduit directement sur l'halogénométhylcétone **6,** dans le cas où X=Br. Ce dernier traité par NaI, subit un échange d'halogène et réagit ensuite sur le sel de sodium du dialkylmalonate substitué pour donner le composé **8.**

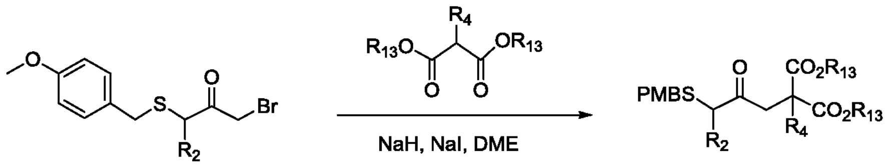


6

8

Cette réaction permet de conserver la configuration du carbone portant le thiol.

**Etape 4**

Après hydrolyse des esters de **8,** par action du TFA (cas où $R_{13}$ est un groupement tert-Butyle) ou par saponification (cas où $R_{13}$ est un groupement Méthyle ou Ethyle), une décarboxylation à reflux dans le toluène, par exemple, conduit au composé **9.**

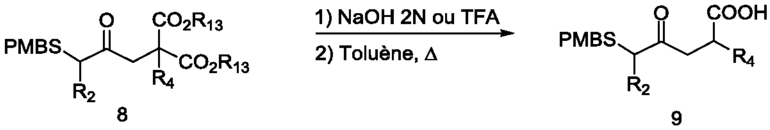


8

9

**Etape 5**

La déprotection du thiol présent sur le composé **9** s'effectue en 2 temps par action de DTNP (2,2'-dithiobis(5-nitropyridine)) dans l'acide trifluoroacétique suivi d'une réaction avec le TCEP (tris(2-carboxyethyl)phosphine) (Harris K.M. et al. (2007) J. Pept. Sci. (2), 81-93) ou bien directement par chauffage dans l'acide trifluoroacétique à 50°C en présence d'anisole pour donner l'acide mercaptoalcanoïque **10.**

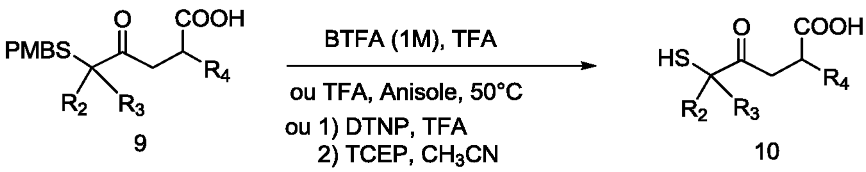


9

10

Les composés **10** dans lesquels $R_2$ = $R_3$ = alkyl, R = H et $R_5$ = OH peuvent être obtenus par réaction entre l'acide bromoacétique et le 4-methoxy benzyl mercaptan pour donner les chlorométhylcétones **6,** suivi par la double alkylation et la transformation en chlorométhylcétone **6** comme décrit précédemment.

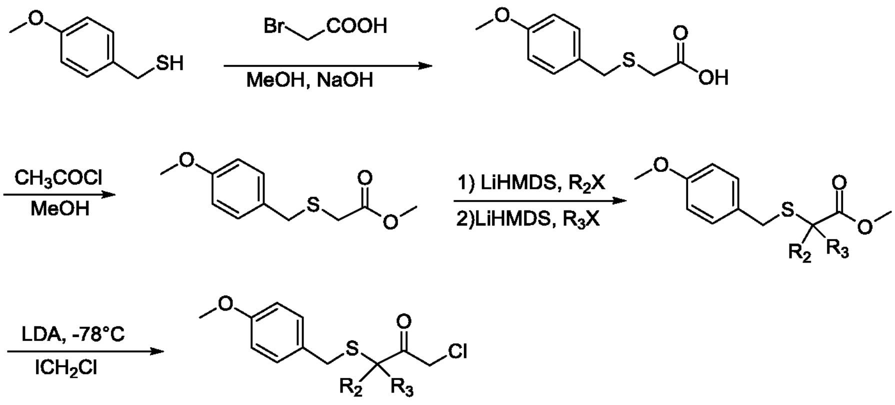


6

Les composés **10** dans lesquels $R_2$ et $R_3$ forment un cycle peuvent être obtenus par préparation de la chlorométhylcétone **6** directement à partir de l'ester correspondant (méthylique par exemple) par alkylation à l'aide du disulfure

du 4-methoxy benzyl mercaptan (ou à partir d'une autre activation de ce thiol) et transformation en chlorométhylcétone **6.**

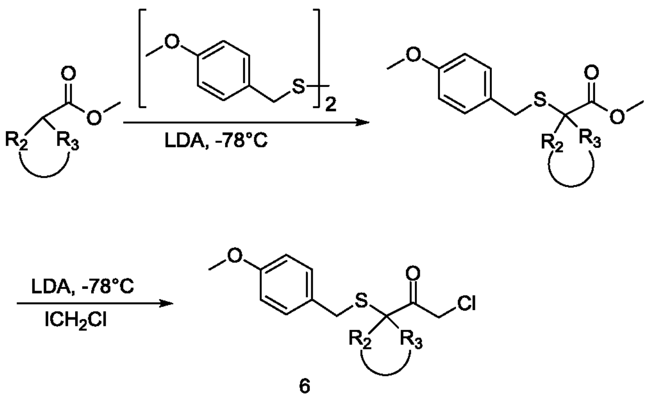


6

Alternativement, la chlorométhylcétone géminée cyclique **6** peut être préparée directement à partir de l'ester correspondant (méthylique par exemple) par alkylation à l'aide de 4-methoxy benzylmercaptan activée et transformation en bromométhylcétone **6** à l'aide d'une solution de $TMSN_2$.

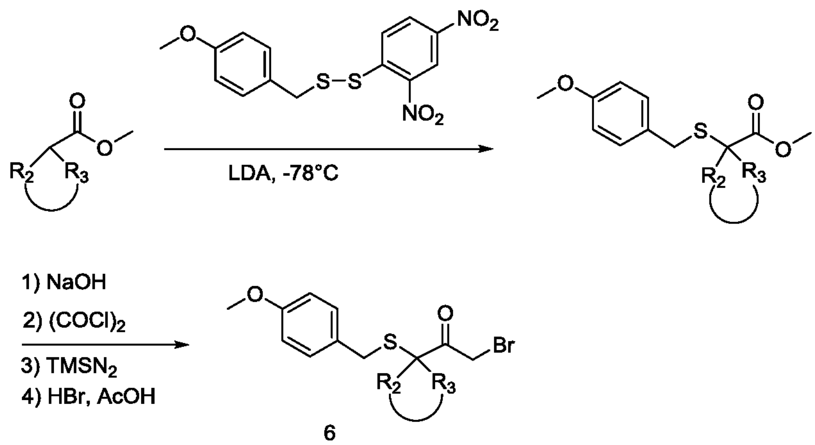


6

La suite de la synthèse s'effectue comme décrit précédemment.

Dans le cas où $R_4$ est un 4-Bromobenzyl, le composé **9** peut subir une réaction de Suzuki pour introduire un aromatique sur le benzyl.

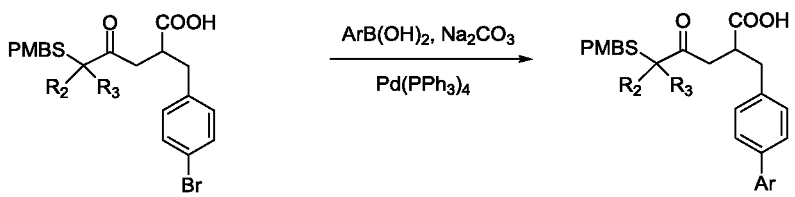


Les composés **9,** lorsque $R_3$ = H, présentent 2 centres d'asymétrie et sont constitués de 4 stéréoisomères. Lorsque $R_2$ = $R_3$ = alkyl ou cycle, les composés **9** présentent un seul centre d'asymétrie et sont donc un mélange de 2 stéréoisomères.

Les composés **10** sont obtenus après déprotection de **9.**

Dans le cas où les composés **9** sont chiraux, ils peuvent être séparés par précipitation sélective avec des amines chirales comme l'$\alpha$-methylbenzylamine ou la noréphédrine ou par HPLC sur colonne chirale.

## EXEMPLES

L'invention sera encore illustrée sans aucunement n'être limitée par les exemples ci-après.

## 1. Synthèse des inhibiteurs mixtes NEP-APN

### 1.1 Synthèse des inhibiteurs de NEP

**1.1.1 Etape 1:** synthèse des acides (*R*)-2-Bromo carboxyliques

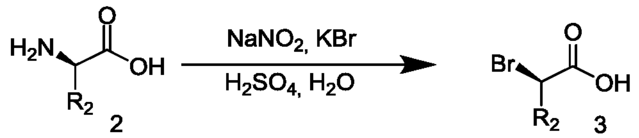

2 3

L'acide aminé de configuration (R)- (39.3 mmol) est solubilisé dans 50 mL d'eau. A 0°C, sont respectivement ajoutés KBr (3.5 eq, 31.8g) puis $H_2SO_4$ (7.73mL) goutte à goutte, en contrôlant que la température reste inférieure à 5°C. Le mélange est refroidi à -10°C et $NaNO_2$ (1.3 eq, 3.59g) solubilisé dans 17 mL d'eau est ajouté goutte à goutte. Le mélange est agité 2h à -5°C.

Après retour à température ambiante, le mélange est extrait par $CH_2Cl_2$ (2*50mL). La phase organique est lavée par $H_2O$, NaCl sat., séchée sur $Na_2SO_4$ pour donner le produit attendu de configuration (R).

**3a** $R_2$ = $CH_2Ph$: huile jaune claire; (Rdt: 50%); Rf ($CH_2Cl_2$/MeOH): 0.62 RMN ($CDCl_3$, 200 MHz): 3.15-3.40 (2H, dd); 4.69 (1H, m); 7.20-7.40 (5H, m)
**3b** $R_2$ = $CH_2CH(CH_3)_2$: huile; (Rdt: 82.5%); Rf ($CH_2Cl_2$/MeOH): 0.49 RMN ($CDCl_3$, 200 MHz): 0.90-1.0 (6H, m); 1.55 (2H, m); 2.40 (1H, m); 4.29 (1H, d)
**3c** $R_2$ = $CH_2$(4-Br-Ph): huile jaune claire; (Rdt: 50%); Rf ($CH_2Cl_2$/MeOH): 0.62 RMN ($CDCl_3$, 200 MHz): 3.15-3.40 (2H, dd); 4.70 (1H, m); 7.20-7.40 (4H, m)
**3d** $R_2$ = $CH_2$(4-Ph-Ph): huile jaune claire; (Rdt: 60%); Rf ($CH_2Cl_2$/MeOH): 0.7 RMN ($CDCl_3$, 200 MHz): 3.15-3.40 (2H, dd); 4.70 (1H, m); 7.20-7.60 (9H, m)

Synthèse des acides (*S*)-2-(4-methoxybenzylthio)carboxyliques

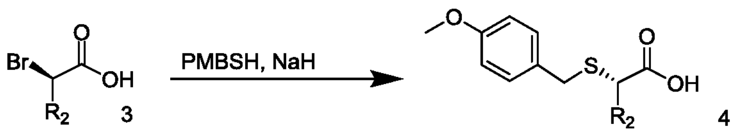

3 4

Sous atmosphère inerte, le 4-méthoxybenzyl mercaptan (4.2 mL; 30.06 mmol, 1 eq) est solubilisé dans 70 mL de THF anhydre et 1.1 éq de NaH 60% (1.33 g; 33.07 mmol) sont ajoutés. Le mélange est agité 15 min à température ambiante puis le dérivé bromé **3** (1 eq, 30.06 mmol) solubilisé dans 30 mL de THF est ajouté goutte à goutte à l'aide d'une ampoule à brome. Le mélange est agité durant la nuit à température ambiante. Le mélange est évaporé à sec puis est repris par AcOEt. La phase organique est lavée par $H_2O$, NaCl sat., séchée par $Na_2SO_4$, évaporée sous pression réduite pour donner le produit brut. Celui-ci est purifié par chromatographie sur silice avec CHex/AcOEt 5/5 comme système d'élution pour donner le composé **4** de configuration (S) sous forme d'huile.

**4a** $R_2$ = $CH_2Ph$: huile; (Rdt: 40%); Rf ($CH_2Cl_2$/MeOH/ 9/1): 0.5
RMN ($CDCl_3$, 200 MHz): 2.80-3.10 (2H, m); 3.68 (2H, s); 3.76 (3H, s); 4.53 (1H, t); 6.84 (2H, d); 7.09-7.49 (7H, m)
**4b** $R_2$ = $CH_2CH(CH_3)_2$: huile; (Rdt: 26%); Rf (CHex/AcOEt): 0.65
RMN ($CDCl_3$, 200 MHz): 0.90-1.0 (6H, m); 1.55 (2H, m); 2.25 (1H, m); 3.40 (1H, d); 3.70 (2H, s); 3.90 (3H, s); 6.8-6.9 (4H, m)
**4c** $R_2$ = $CH_2$(4-Br-Ph): huile; (Rdt: 40%); Rf ($CH_2Cl_2$/MeOH/ 9/1): 0.5
RMN ($CDCl_3$, 200 MHz): 3.0-3.30 (2H, m); 3.60 (1H, q); 3.70 (2H, s); 3.90 (3H, s); 6.80-7.20 (8H, m)
**4d** $R_2$ = $CH_2$(4-Ph-Ph): huile; (Rdt: 50%); Rf ($CH_2Cl_2$/MeOH/ 9/1): 0.6
RMN ($CDCl_3$, 200 MHz): 3.0-3.30 (2H, m); 3.60 (1H, q); 3.70 (2H, s); 3.90 (3H, s); 6.80-7.2 (13H, m)

**1.1.2 Etape 2:**

Procédé 1:

Méthode 1: synthèse de la diazocétone à partir de l'anhydride mixte

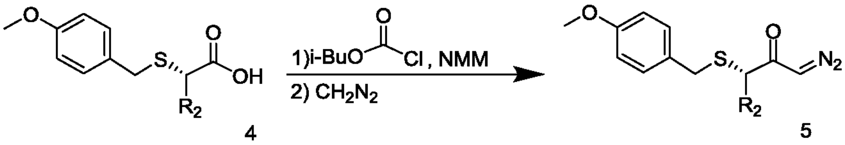


4 5

A une solution d'acide **4** (18.5 mmol) dans 20 mL de THF sec, sous atmosphère inerte, sont ajoutés successivement à -20°C, la N-Méthyl morpholine (2.15 mL; 1.05 eq) et iBuOCOCl (2.52 mL; 1.05 eq). Le mélange est agité pendant 5-10 min à -20°C puis le précipité est filtré sur célite et lavé par 20 mL THF.

La solution de $CH_2N_2$ dans l'éther (2.5 eq) (préalablement préparée à partir de Diazald® et de KOH dans le carbitol), est transférée à la solution d'ester activé à 0°C. La solution devient jaune. Le mélange est agité 2h à température ambiante.

Méthode 2: synthèse de la diazocétone à partir du chlorure d'acide

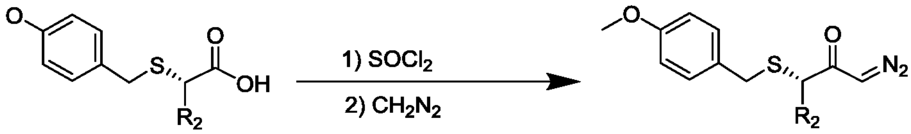


4 5

L'acide **4** (14.5 mmol) est solubilisé dans 23 mL de $CH_2Cl_2$ anhydre. $SOCl_2$ (1.5 eq; 21.75 mmol) est ajouté à température ambiante et le mélange est porté 2h au reflux, sous atmosphère inerte. Le mélange est ensuite évaporé à sec pour donner une huile brunâtre. Le produit est solubilisé dans le THF anhydre à raison de 5 mmol/mL.

La solution de $CH_2N_2$ (2.5 eq) dans l'éther, préalablement préparée est transférée à la solution de chlorure d'acide à 0°C. La solution devient jaune. Le mélange est agité 2h à température ambiante, sous atmosphère inerte.

Synthèse de la chlorométhylcétone

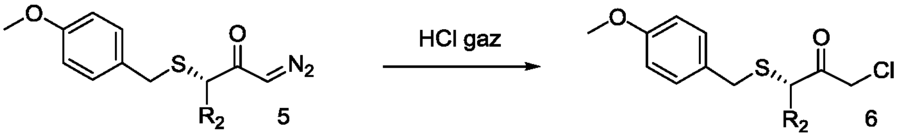


5 6

La solution du composé **5** est placée, sous atmosphère inerte, dans un tricol maintenu à 0°C. Le mélange est saturé en HCl, par bullage à 0°C.

Au bout de 30 min, le solvant et l'excès de HCl sont évaporés sous pression réduite. Le produit est repris dans AcOEt (150 mL) puis est lavé par $NaHCO_3$ 10%, $H_2O$ et séché sur $Na_2SO_4$ pour donner le produit à l'état brut. Ce dernier est utilisé tel quel sans purification pour l'étape suivante.

Procédé 2: synthèse à partir de l'ester méthylique

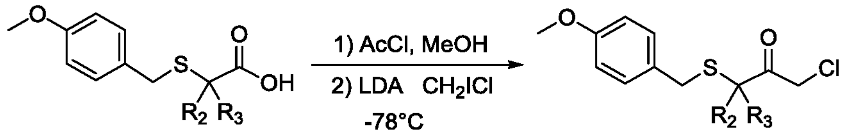


4 6

Le chlorure d'acétyle (3eq; 2mL) est ajouté goutte à goutte à 0°C, sous gaz inerte, à une solution d'acide **4** (9.1 mmol) dans 50 mL de MeOH anhydre. Le mélange est agité pendant la nuit à température ambiante. Le mélange est concentré sous pression réduite, repris dans MTBE (méthyl-tert-butyl éther) (200mL). La phase organique est lavée par $NaHCO_3$ 10% (100mL), $H_2O$ (100mL) et NaCl sat. (100mL). La phase organique est séchée sur $Na_2SO_4$ puis est concentrée sous pression réduite pour donner l'ester méthylique à l'état brut. Ce dernier est chromatographié par système flash sur gel de silice.

Une solution de LDA (5 éq) dans le THF (55 mL), fraichement préparée à partir de BuLi 1,6M in hexane (15 mL) et de diisopropylamine (3,4 mL), est additionnée goutte-à-goutte en 30 min à l'ester méthylique (4,42 mmol) et au chloroiodométhane (1,3 mL; 4 éq) en solution dans 25 mL de THF. La température interne de la réaction est maintenue en dessous de -70°C pendant l'addition et à -75°C pendant 10 min. Une solution d'acide acétique (6mL dans 44 mL de THF) est ajoutée en gardant la température inférieure à -65°C, pour neutraliser le milieu. Le mélange est alors extrait par AcOEt. La phase organique est lavée par $NaHCO_3$ 10%, acide citrique 10%, NaCl sat., séchée sur $Na_2SO_4$, puis concentrée sous pression réduite pour donner le produit brut qui est utilisé tel quel pour l'étape suivante.

**6a** $R_2 = CH_2Ph$; $R_3$ = H: huile orangée; (Rdt: 93.0%); Rf (CHex/AcOEt 6/4): 0.73 HPLC: Kromasil C18 $CH_3CN$(0.1% TFA)/$H_2O$ (0.1% TFA) 60/40 Rt: 19.18 min RMN ($CDCl_3$, 200MHz): 2.85 (1H, dd); 3.2 (1H, dd); 3.55 (1H, d); 3.6 (2H, d); 3.7 (3H, s); 4.1 (2H, d); 6.7 (2H, d); 7.2-7.4 (7H, m)

**6b** $R_2 = CH_2CH(CH_3)_2$; $R_3$ = H: huile orange; (Rdt: 94.0%); Rf (CHex/AcOEt 5/5): 0.68 RMN ($CDCl_3$, 200MHz): 0.90-1.0 (6H, m); 1.55 (2H, m); 2.25 (1H, m); 3.40 (1H, d); 3.70 (2H, s); 3.90 (3H, s); 4.25 (2H, d); 6.80 (2H, d); 7.15 (2H, d)

**6c** $R_2 = CH_2$(4-Br-Ph); $R_3$ = H: huile orangée; (Rdt: 85.0%); Rf (CHex/AcOEt 6/4): 0.80 RMN ($CDCl_3$, 200MHz): 2.85 (1H, dd); 3.2 (1H, dd); 3.55 (1H, d); 3.6 (2H, d); 3.7 (3H, s); 4.1 (2H, s); 6.7 (2H, d); 7.2-7.4 (6H, m)

**6d** $R_2 = CH_2$(4-Ph-Ph); $R_3$ = H: huile orangée; (Rdt: 90.0%); Rf (CHex/AcOEt 6/4): 0.73 RMN ($CDCl_3$, 200MHz): 2.85 (1H, dd); 3.2 (1H, dd); 3.55 (1H, d); 3.6 (2H, d); 3.7 (3H, s); 4.1 (2H, s); 6.7 (2H, d); 7.2-7.5 (11H, m)

Synthèse des chlorométhylcétones géminées

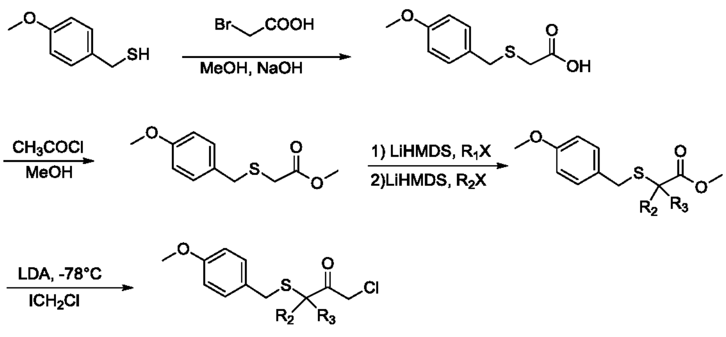


6

L'acide bromoacétique (10g, 72mmol) est mis en solution, sous atmosphère inerte, dans 50 mL de MeOH. 11 mL (1.1éq) de 4-methoxybenzyl mercaptan sont ajoutés à 4°C et une solution de soude alcoolique (6.4g de NaOH (2.2 eq) en solution dans 100 mL de MeOH) sont ajoutés goutte à goutte. Le mélange est agité 40 min à température ambiante. Le solvant est évaporé sous pression réduite. Le produit est repris dans $Et_2O$ (200 mL) et 350 mL de $NaHCO_3$ 10%. La phase aqueuse est acidifiée jusqu'à pH=1 puis est extraite par 350 mL de $Et_2O$. La phase organique est lavée par $H_2O$ (100mL), NaCl sat (100mL) et est séchée sur $Na_2SO_4$ pour donner 15g d'un solide blanc brut (Rdt : 98%) qui est utilisé tel quel pour l'étape suivante.

HPLC: Atlantis T3, $CH_3CN$(0.1 % TFA)/$H_2O$ (0.1% TFA) Gradient 10-90% 15 min, Rt = 10.64 min

RMN ($CDCl_3$, 200MHz): 3.0 (2H, s); 3.75 (3H, s); 3.75 (2H, s); 6.80 (2H, d); 7.20 (2H, d)

Le chlorure d'acétyle (1.5eq; 7.6mL; 106 mmol) est ajouté goutte à goutte à 4°C, sous atmosphère inerte, à une solution de l'acide précédent (70.8 mmol) dans 150 mL de MeOH anhydre. Le mélange est agité durant la nuit à température ambiante. Le mélange est concentré sous pression réduite, repris dans MTBE (méthyl-tert-butyl éther) (350mL). La phase organique est lavée par HCl 0.5N (2*100mL), $NaHCO_3$ 10% (2*100mL), $H_2O$ (100mL) et NaCl sat. (100mL). La phase organique est séchée sur $Na_2SO_4$ puis est concentrée sous pression réduite pour donner l'ester méthylique.
HPLC: Atlantis T3, $CH_3CN$(0.1% TFA)/$H_2O$ (0.1% TFA) Gradient 50-90% 10 min, Rt = 5.24min
RMN ($CDCl_3$, 200MHz): 3.1 (2H, s); 3.75 (3H, s); 3.83 (2H, s); 3.85 (3H, s); 6.85 (2H, d); 7.30 (2H, d).

Une solution de LiHMDS 1M THF (4.4mL; 1eq) est ajoutée goutte à goutte, sous atmosphère inerte, à -78°C, à une solution de l'ester précédent (1g; 4.4 mmol; 1eq) solubilisé dans 5mL de THF anhydre. Le mélange est agité 1h à -78°C puis la solution du dérivé RX (1 eq) est ajoutée sous atmosphère inerte à -78°C. Le mélange ramené à température ambiante est agité pendant 3h. Le mélange est à nouveau refroidit à -78°C et 1eq de LiHMDS est ajouté suivi par 1.5eq de RX. Le mélange ramené à température ambiante est agité pendant 4h. Le mélange est alors partitionné entre 200mL HCl 1N et 300mL AcOEt. La phase organique est séchée sur $Na_2SO_4$ puis est concentrée sous pression réduite pour donner le produit brut qui purifié par chromatographie sur gel de silice.
$R_2 = CH_3$, $R_3 = CH_3$: huile (Rdt: 44%)
HPLC: Atlantis T3, $CH_3CN$(0.1% TFA)/$H_2O$ (0.1% TFA) Gradient 50-90% 10 min, Rt = 7.39 min
RMN ($CDCl_3$, 200MHz): 1.60 (6H, s); 3.70 (3H, s); 3.80 (2H, s); 3.80 (3H, s); 6.85 (2H, d); 7.25 (2H, d)
$R_2 = C_2H_5$, $R_3 = C_2H_5$: huile (Rdt: 55%)
HPLC: Atlantis T3, $CH_3CN$(0.1% TFA)/$H_2O$ (0.1% TFA) Gradient 50-90% 10 min, Rt = 9.72 min
RMN ($CDCl_3$, 200MHz): 0.90 (6H, t); 1.85 (4H, m); 3.68 (2H, s); 3.70 (3H, s); 3.80 (3H, s); 6.82 (2H, d); 7.22 (2H, d)

La chlorométhylcétone est synthétisée comme décrit en 4b

**6e** $R_2 = CH_3$, $R_3 = CH_3$: huile ambrée
HPLC: Atlantis T3, $CH_3CN$(0.1 % TFA)/$H_2O$ (0.1% TFA) Gradient 50-90% 10 min, Rt = 7.91 min
RMN ($CDCl_3$, 200MHz): 1.60 (6H, s); 3.55 (1H, d); 3.60 (2H, s); 3.80 (3H, s); 4.45 (2H, s); 6.7 (2H, d); 7.2 (2H, d)
**6f** $R_2 = C_2H_5$, $R_3 = C_2H_5$: huile ambrée
HPLC: Atlantis T3, $CH_3CN$(0.1 % TFA)/$H_2O$ (0.1% TFA) Gradient 50-90% 10 min, Rt = 10.04 min
RMN ($CDCl_3$, 200MHz): 0.85 (6H, t); 1.80 (4H, q); 3.55 (1H, d); 3.60 (2H, s); 3.80 (3H, s); 4.45 (2H, s); 6.7 (2H, d); 7.2 (2H, d)

<u>Synthèse des chlorométhylcétones cycliques</u>

Procédé 1 :

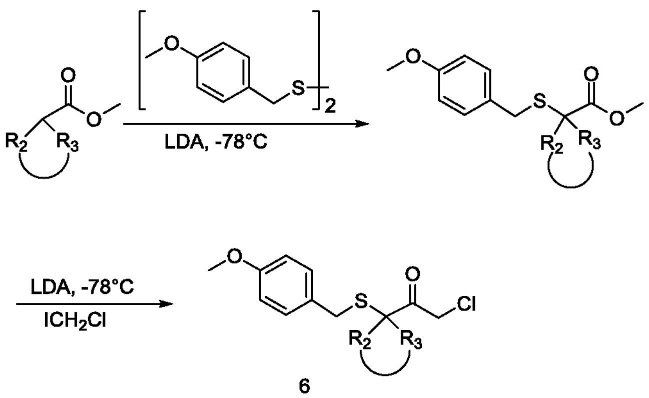


6

A une solution dans le THF (9mL), sous atmosphère inerte, de DIPA (diisopropylethylamine) (3 mmol; 1.3 eq; 420μL) est introduit à -10°C, la solution 2.5M de BuLi dans l'hexane (2.77 mmol; 1.1mL; 1.2eq). Le mélange est agité 1h à 0°C. Cette solution de LDA, fraichement préparée, est ajoutée goutte à goutte à une solution d'ester méthylique d'acide cyclopentanoïque dans 5mL de THF à -55°C. Le mélange est agité 1h à -55°C sous atmosphère inerte. Le HMPA (hexylmethylphophoramide) (3.46 mmol; 1.5eq; 610μL) est ajouté et le mélange est agité pendant 10 min à la même température. Une solution de disulfure de 4-methoxybenzyl mercaptan (3 mmol; 1.3 eq; 920 mg) dans 12mL de THF est alors ajoutée goutte à goutte à -55°C. Après retour à température ambiante, le mélange est agité durant la nuit.

Le mélange est partitionné entre 10mL $NH_4Cl$ sat. et 20mL AcOEt. La phase organique est lavée par $NH_4Cl$ sat. (2*10 mL), NaCl sat (2*15 mL), séchée sur $Na_2SO_4$ puis est concentrée sous pression réduite pour donner le produit brut qui est purifié par chromatographie sur gel de silice.
$C(R_2R_3)$ = Cyclopentyl: huile (Rdt: 40%)
HPLC: Atlantis T3, $CH_3CN$(0.1 % TFA)/$H_2O$ (0.1% TFA) Gradient 30-90% 10 min, Rt = 9.68 min
RMN ($CDCl_3$, 200MHz): 1.60-2.40 (8H, m); 3.67 (3H, s); 3.77 (2H, s); 3.80 (3H, s); 6.83 (2H, d); 7.24 (2H, d)
La chlorométhylcétone est synthétisée comme décrit en 4b

**6g** $C(R_2)(R_3)$ = Cyclopentyl: huile ambrée
HPLC: Atlantis T3, $CH_3CN$(0.1 % TFA)/$H_2O$ (0.1% TFA) Gradient 60-90% 10 min, Rt = 6.47 min
RMN ($CDCl_3$, 200MHz): 1.60-1.80 (8H, m); 3.54 (2H, s); 3.80 (3H, s); 4.47 (2H, s); 6.78 (2H, d); 7.22 (2H, d)
**6h** $C(R_2)(R_3)$ = Cyclohexyl: huile ambrée
HPLC: Atlantis T3, $CH_3CN$(0.1 % TFA)/$H_2O$ (0.1% TFA) Gradient 70-90% 10 min, Rt = 8.11 min
RMN ($CDCl_3$, 200MHz): 1.40-2.20 (10H, m); 3.45 (2H, s); 3.80 (3H, s); 4.40 (2H, s); 6.80 (2H, d); 7.20 (2H, d)

<u>Procédé 2:</u>

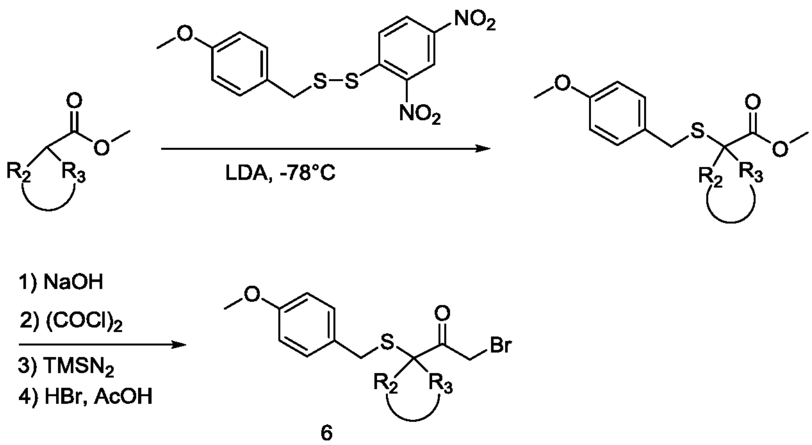


6

A une solution dans le THF (10mL), sous atmosphère inerte, de DIPA (diisopropylethylamine) (16.7 mmol; 1.2 eq; 2.34mL) est introduit à -10°C, la solution 2.5M de BuLi dans l'hexane (16 mmol; 6.4mL; 1.15eq). Le mélange est agité 1h à 0°C. Cette solution de LDA, fraichement préparée, est ajoutée goutte à goutte à une solution d'ester méthylique d'acide phenylcyclopentanoïque dans 5mL de THF et HMPA (hexylmethylphophoramide) (1.0eq; 2.5mL) à -78°C. Le mélange est agité 1h à -78°C sous atmosphère inerte. Le thiol de 4-methoxybenzyl mercaptan activé (18 mmol; 1.3 eq; 6.37 mg) est ajouté à l'état solide à -78°C. Le mélange est agité 1h30 à -78°C. Le mélange est partitionné entre 200mL HCl 1N et 200mL AcOEt. La phase organique est diluée avec 200 mL AcOEt, lavée par NaCl sat (200 mL), séchée sur $Na_2SO_4$ puis est concentrée sous pression réduite pour donner le produit brut qui est purifié par chromatographie sur gel de silice.
$C(R_2R_3)$ = PhénylCyclopentyl (Indanyl): solide blanc (Rdt: 25%)
HPLC: Atlantis T3, $CH_3CN$(0.1 % TFA)/$H_2O$ (0.1% TFA) Gradient 50-90% 10 min, Rt = 9.59 min
RMN ($CDCl_3$, 200MHz): 2.90-3.70 (6H, m); 3.55 (3H, s); 3.65 (3H, s); 6.60-7.10 (8H, m)
Le produit de l'étape précédente (1.13g; 3.44 mmol) est solubilisé dans 14mL d'un mélange THF/MeOH. 14mL de NaOH 2N sont ajoutés et le mélange est agité 3h à température ambiante. Le mélange est dilué par 40mL $H_2O$. Le mélange est concentré sous pression réduite. La phase aqueuse est acidifiée par HCl 1N puis est extraite par MTBE (3 x 100mL), lavée par NaCl sat (100 mL), séchée sur $Na_2SO_4$ puis est concentrée sous pression réduite pour donner un solide blanc (Rdt : 98%).
HPLC: Atlantis T3, $CH_3CN$(0.1 % TFA)/$H_2O$ (0.1% TFA) Gradient 50-90% 10 min, Rt = 6.50 min
Le chlorure d'oxalyle (2 mmol; 1.5eq; 177$\mu$L) est ajouté goutte à goutte, sous atmosphère inerte, à 0°C, à une solution de l'acide précédent (1.38 mmol; 434mg) dans 10mL $CH_2Cl_2$, en présence de 20$\mu$L DMF (0.2 eq). Le mélange est laissé revenir à température ambiante puis est agité 30 min à température ambiante.
HPLC: Atlantis T3, $CH_3CN$(0.1 % TFA)/$H_2O$ (0.1% TFA) Gradient 70-90% 10 min, Rt = 6.70 min.
Après évaporation sous pression réduite, le chlorure d'acide est repris, sous atmosphère inerte, dans $CH_3CN$

anhydre (5mL) et $TMSCHN_2$ (1M dans $Et_2O$) (1.5eq; 1mL) est additionné goutte à goutte à 0°C. Le mélange est laissé revenir à température ambiante puis est agité 1h30. Le mélange est ensuite piégé par 300µL (1.65 mmol; 1.2 eq) HBr 33% dans l'acide acétique. Le mélange est agité 15 min à température ambiante. Le mélange est concentré sous pression réduite puis est repris par MTBE (200mL). La phase organique est lavée par $NaHCO_3$ 10% (100mL), NaCl sat (50 mL), séchée sur $Na_2SO_4$ puis est concentrée sous pression réduite pour donner le composé 6h sous forme d'une huile marron (Rdt: 83%).

**6h** $C(R_2R_3)$ = Phénylcyclopentyl (Indanyl): solide blanc
HPLC: Atlantis T3, $CH_3CN$(0.1 % TFA)/$H_2O$ (0.1% TFA) Gradient 70-90% 10 min, Rt = 5.30 min
RMN ($CDCl_3$, 200MHz): 2.90-3.70 (6H, m); 3.65 (3H, s); 4.15 (2H, s); 6.60-7.10 (8H, m)

### 1.1.3 Etape 3

Procédé 1

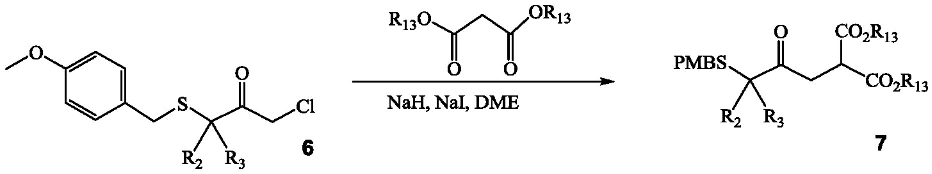


6

7

Sous atmosphère inerte, 980 mg de NaH à 60% (1 eq) sont ajoutés au dialkylmalonate (1 eq) dans 25mL (1mL/mmol) de DME (1,2-dimethoxyethane). Le mélange est agité 1h à température ambiante.

Un mélange de chlorométhylcétone **6** (24.42 mmol) et de NaI (24.42 mmol, 3.66g, 1 eq) dans 50mL de DME est agité à température ambiante pendant 15 min et est ensuite ajouté à une solution fraîchement préparée de sel de sodium de dialkylmalonate. Le mélange est agité 4h à température ambiante.

A la fin de la réaction, le solvant est évaporé sous pression réduite. Le produit est repris dans le dichlorométhane. La phase organique est lavée à l'eau et séchée sur $Na_2SO_4$. Le produit est chromatographié sur colonne de silice avec CHex/AcOEt 9/1 comme système d'élution.

**7a1** $R_2 = CH_2Ph$, $R_3$ = H, $R_{13} = CH_2CH_3$: huile orangée; (Rdt: 60 %); Rf (CHex/AcOEt 9/1): 0.16
HPLC: Kromasil C18 $CH_3CN/H_2O$ (0.1% TFA) 80/20 Rt: 6.57 min
RMN ($CDCl_3$, 200MHz): 1.30 (6H, t); 2.70-3.40 (4H, m); 3.45 (1H, t); 3.50 (2H, d); 3.60 (1H, t); 3.65 (3H, s); 4.15 (4H, q); 6.75 (2H, d); 7.2-7.4 (7H, m)
**7a2** $R_2 = CH_2Ph$, $R_3$ = H, $R_{13}$ = tBu: huile orangée; (Rdt: 62 %); Rf (CHex/AcOEt 9/1): 0.36
HPLC: Kromasil C18 $CH_3CN/H_2O$ (0.1% TFA) 85/15 Rt: 15.51 min
RMN ($CDCl_3$, 200MHz): 1.40 (18H, s); 2.70-3.40 (4H, m); 3.45 (1H, t); 3.50 (2H, d); 3.60 (1H, t); 3.65 (3H, s); 6.75 (2H, d); 7.2-7.4 (7H, m)
**7b** $R_2 = CH_2CH(CH_3)_2$, $R_3$ = H, $R_{13}$ = tBu: huile orangée; (Rdt: 30 %); Rf (CHex/AcOEt 9/1): 0.49
HPLC: Kromasil C18 $CH_3CN/H_2O$ (0.1% TFA) 90/10 Rt: 6.49 min
RMN ($CDCl_3$, 200MHz): 0.90-1.0 (6H, m), 1.40 (18H, s); 1.55 (2H, m); 2.15 (1H, m); 3.19 (2H, m); 3.40 (2H, m); 3.50 (2H, d); 3.80 (3H, s); 6.75 (2H, d); 7.2 (2H, d)
**7c** $R_2 = CH_2$(4-Br-Ph), $R_3$ = H, $R_{13}$ = tBu: huile orangée; (Rdt: 62 %); Rf (CHex/AcOEt 9/1): 0.36
HPLC: Kromasil C18 $CH_3CN/H_2O$ (0.1% TFA) 85/15 Rt: 15.51 min
RMN ($CDCl_3$, 200MHz): 1.40 (18H, s); 2.70-3.40 (4H, m); 3.45 (1H, t); 3.50 (2H, d); 3.60 (1H, t); 3.65 (3H, s); 6.75 (2H, d); 7.2-7.4 (6H, m)
**7d** $R_2 = CH_2$(4-Ph-Ph), $R_3$ = H, $R_{13}$ = tBu: huile orangée; (Rdt : 60 %); Rf (CHex/AcOEt 9/1): 0.36
HPLC: Kromasil C18 $CH_3CN/H_2O$ (0.1% TFA) 85/15 Rt: 16.71 min
RMN ($CDCl_3$, 200MHz): 1.40 (18H, s); 2.70-3.40 (4H, m); 3.45 (1H, t); 3.50 (2H, d); 3.60 (1H, t); 3.65 (3H, s); 6.75 (2H, d); 7.2-7.5 (11H, m)
**7e** $R_2 = CH_3$, $R_3 = CH_3$, $R_{13}$ = Et (Rdt: 40%)
HPLC: Atlantis T3, $CH_3CN$(0.1% TFA)/$H_2O$ (0.1% TFA) Gradient 70-90% 10 min, Rt = 4.87 min
RMN ($CDCl_3$, 200MHz): 1.30 (6H, t); 1.55 (6H, s); 3.40 (2H, d); 3.55 (2H, s); 3.75 (1H, t); 3.82 (3H, s); 4.25 (4H, q); 6.85 (2H, d); 7.25 (2H, d)
**7f** $R_2 = C_2H_5$, $R_3 = C_2H_5$, $R_{13}$ = Et (Rdt: 30%)

HPLC: Atlantis T3, $CH_3CN$(0.1% TFA)/$H_2O$ (0.1% TFA) Gradient 50-90% 10 min puis 90-50%, Rt = 11.56 min
RMN ($CDCl_3$, 200MHz): 0.80 (6H, t); 1.20 (6H, t); 1.75 (4H, m); 3.30 (2H, s); 3.38 (2H, d); 3.65 (1H, t); 3.72 (3H, s); 4.15 (4H, q); 6.72 (2H, d); 7.15 (2H, d)
**7g** $C(R_2)(R_3)$ = Cyclopentyl, $R_{13}$: Et (Rdt: 24%)
HPLC: Atlantis T3, $CH_3CN$(0.1% TFA)/$H_2O$ (0.1% TFA) Gradient 60-90% 10 min, Rt = 8.04 min
RMN ($CDCl_3$, 200MHz): 1.29 (6H, t); 1.60-2.30 (8H, m); 3.39 (2H, d); 3.52 (2H, s); 3.74 (1H, t); 3.79 (3H, s); 4.22 (4H, q); 6.83 (2H, d); 7.20 (2H, d)
**7h** $C(R_2)(R_3)$ = Cyclohexyl, $R_{13}$: Et (Rdt: 33%)
HPLC: Atlantis T3, $CH_3CN$(0.1% TFA)/$H_2O$ (0.1% TFA) Gradient 70-90% 10 min, Rt = 7.76 min
RMN ($CDCl_3$, 200MHz): 1.29 (6H, t); 1.60-2.30 (10H, m); 3.45 (2H, d); 3.50 (2H, s); 3.75 (1H, t); 3.80 (3H, s); 4.25 (4H, q); 6.85 (2H, d); 7.20 (2H, d)
**7i** $C(R_2)(R_3)$ = Phénylcyclopentyl (Indanyl), $R_{13}$: Et (Rdt: 80%)
HPLC: Atlantis T3, $CH_3CN$(0.1% TFA)/$H_2O$ (0.1% TFA) Gradient 70-90% 10 min, Rt = 6.57 min
RMN ($CDCl_3$, 200MHz): 1.20 (6H, t); 2.90-3.60 (9H, m); 3.65 (3H, s); 4.15 (4H, q); 6.60-7.10 (8H, m)

Alkylation du malonate

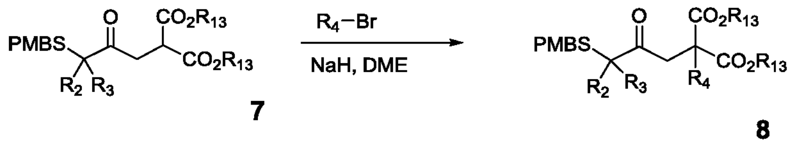


**7** **8**

A une solution du produit **7** dans 15 mL de DME (diméthoxyethane), sont ajoutés 1.5 eq de NaH 60%.
Le mélange est agité 1h à température ambiante puis le dérivé bromé $R_4Br$ (3 eq) est ajouté. Le mélange est agité durant la nuit à température ambiante.
Le solvant est évaporé sous pression réduite puis le mélange est repris par $H_2O$ et AcOEt. La phase organique est lavée par $H_2O$, séchée sur $Na_2SO_4$ puis concentrée sous pression réduite.
Le produit est chromatographié sur silice avec CHex/AcOEt 9/1 comme système d'élution pour donner le produit désiré **8.**

**8a** $R_2 = CH_2Ph$; $R_3$ = H; $R_4 = CH_2$(4-Br-Ph); $R_{13}$ = Et: huile orangée
**8b** $R_2 = CH_2Ph$; $R_3$ = H; $R_4 = CH_2Ph$; $R_{13}$ = tBu: huile orangée
**8c** $R_2 = CH_2CH(CH_3)_2$; $R_3$ = H; $R_4 = CH_2$(4-Ph-Ph); $R_{13}$ = Et: huile orangée
**8d** $R_2 = CH_2CH(CH_3)_2$; $R_3$ = H; $R_4 = CH_2Ph$; $R_{13}$ = Et: huile orangée
**8e** $R_2 = CH_2CH(CH_3)_2$; $R_3$ = H; $R_4 = CH_2$(4-Br-Ph); $R_{13}$ = Et: huile orangée
**8f** $R_2 = CH_2$(4-Br-Ph); $R_3$ = H; $R_4 = CH_2Ph$; $R_{13}$ = tBu: huile orangée
**8g** $R_2 = CH_2$(4-Br-Ph); $R_3$ = H; $R_4 = CH_2$(4-Br-Ph); $R_{13}$ = tBu: huile orangée
**8h** $R_2 = CH_2$(4-Ph-Ph); $R_3$ = H; $R_4 = CH_2$(4-Br-Ph); $R_{13}$ = tBu: huile orangée
**8i** $R_2 = CH_3$; $R_3 = CH_3$; $R_4 = CH_2$(4-Br-Ph); $R_{13}$ = Et: huile orangée
**8j** $R_2 = C_2H_5$; $R_3 = C_2H_5$; $R_4 = CH_2$(4-Br-Ph); $R_{13}$ = Et: huile orangée
**8k** $C(R_2)(R_3)$ = Cyclopentyl; $R_4 = CH_2$(4-Br-Ph); $R_{13}$ = Et: huile orangée
**8l** $C(R_2)(R_3)$ = Cyclohexyl; $R_4 = CH_2$(4-Br-Ph); $R_{13}$ = Et: huile orangée
**8m** $C(R_2)(R_3)$ = Phénylcyclopentyl (Indanyl); $R_4 = CH_2$(4-Br-Ph); $R_{13}$ = Et: huile orangée

**1.1.4** **Etape 4**

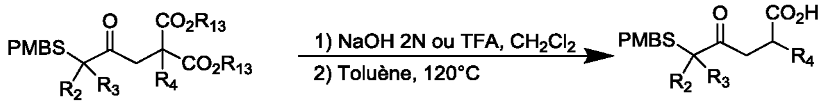


**8** **9**

Lorsque $R_{13}$ est un groupement Méthyle ou Ethyle, le composé **8** (0.585 mmol) est dissous dans 10 mL de

EtOH et NaOH 2N (6eq) est ajouté. Le mélange est agité durant la nuit à température ambiante. L'éthanol est évaporé sous pression réduite. Le produit est repris dans l'eau et est extrait par $Et_2O$. La phase aqueuse est acidifiée par HCl 3N et est extraite par $Et_2O$. La phase organique est séchée sur $Na_2SO_4$ puis est évaporée sous pression réduite pour donner une huile jaunâtre.

Lorsque $R_{13}$ est un groupement tert-Butyl, le produit **8** est dissous dans 10 mL de $CH_2Cl_2$ et 10 mL de TFA sont ajoutés. Le mélange est agité 2h à température ambiante. Les solvants sont évaporés sous pression réduite. Le produit est repris dans l'eau et est extrait par $Et_2O$. La phase organique est séchée sur $Na_2SO_4$ puis est évaporée sous pression réduite pour donner une huile jaunâtre.

Le produit formé est ensuite solubilisé dans 6 mL de toluène et est chauffé à 150°C pendant 12h.

Le solvant est évaporé sous pression réduite pour donner le composé **9.**

**9a** $R_2$ = $CH_2Ph$; $R_3$ = H; $R_4$ = $CH_2$(4-Br-Ph); huile orangée
**9b** $R_2$ = $CH_2Ph$; $R_3$ = H; $R_4$ = $CH_2Ph$; huile orangée
**9c** $R_2$ = $CH_2CH(CH_3)_2$; $R_3$ = H; $R_4$ = $CH_2$(4-Ph-Ph); huile orangée
**9d** $R_2$ = $CH_2CH(CH_3)_2$; $R_3$ = H; $R_4$ = $CH_2Ph$; huile orangée
**9e** $R_2$ = $CH_2CH(CH_3)_2$; $R_3$ = H; $R_4$ = $CH_2$(4-Br-Ph); huile orangée
**9f** $R_2$ = $CH_2$(4-Br-Ph); $R_3$ = H; $R_4$ = $CH_2Ph$; huile orangée
**9g** $R_2$ = $CH_2$(4-Br-Ph); $R_3$ = H; $R_4$ = $CH_2$(4-Br-Ph); huile orangée
**9h** $R_2$ = $CH_2$(4-Ph-Ph); $R_3$ = H; $R_4$ = $CH_2$(4-Br-Ph); huile orangée
**9i** $R_2$ = $CH_3$; $R_3$ = $CH_3$; $R_4$ = $CH_2$(4-Br-Ph); huile orangée
**9j** $R_2$ = $C_2H_5$; $R_3$ = $C_2H_5$; $R_4$ = $CH_2$(4-Br-Ph); huile orangée
**9k** $C(R_2)(R_3)$ = Cyclopentyl; $R_4$ = $CH_2$(4-Br-Ph); huile orangée
**9l** $C(R_2)(R_3)$ = Cyclohexyl; $R_4$ = $CH_2$(4-Br-Ph); huile orangée
**9m** $C(R_2)(R_3)$ = Phénylcyclopentyl (Indanyl); $R_4$ = $CH_2$(4-Br-Ph); huile orangée

Réaction de Suzuki

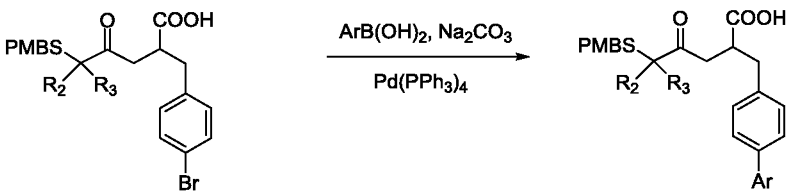


Le composé **9l** (180mg; 0.356mmol) est mis en solution dans 2mL de toluène, sous atmosphère inerte. $Pd(PPh_3)_4$ (11mg; 3% mol) est ajouté et le mélange est agité 5 min à température ambiante. L'acide phénylboronique (46mg; 0.374 mmol; 1.05 eq) est ajouté dans 1 mL MeOH suivi par 500µL de $Na_2CO_3$ 2M. Le mélange est porté au reflux 1h30 puis le mélange est concentré sous pression réduite. Le mélange réactionnel est repris par 30mL $Et_2O$ et 30 mL HCl 1N. La phase organique est lavée par 20mL HCl 1N, 10 mL NaCl sat., séchée sur $Na_2SO_4$ et concentrée sous pression réduite. Le produit est chromatographié sur silice avec Hept/AcOEt 65/35 comme système d'élution pour donner le produit désiré **9n.**

**9n** $C(R_2)(R_3)$ = Cyclohexyl; $R_4$ = $CH_2$(4-Ph-Ph); 75 mg huile orangée (Rdt: 47%) ESI(+): $[M+Na]^+$= 525.2
HPLC: Atlantis T3 $CH_3CN$(0.1% TFA)/$H_2O$ (0.1% TFA) Gradient 70-90% 10 min, Rt = 8.60 min
Les composés suivants sont obtenus selon un protocole identique.
**9o** $C(R_2)(R_3)$ = Cyclopentyl; $R_4$ = $CH_2$(4-Ph-Ph); huile orangée (Rdt: 25%)
ESI(-): $[M-H]^-$= 487.2
HPLC: Atlantis T3 $CH_3CN$(0.1% TFA)/$H_2O$ (0.1% TFA) Gradient 70-90% 10 min, Rt = 6.60 min
**9p** $C(R_2)(R_3)$ = PhénylCyclopentyl (Indanyl); $R_4$ = $CH_2$(4-Ph-Ph); huile orangée (Rdt: 55%)
ESI(-): $[M-H]^-$= 535.2
HPLC: Atlantis T3 $CH_3CN$(0.1% TFA)/$H_2O$ (0.1% TFA) Gradient 70-90% 10 min, Rt = 7.45 min

### 1.1.5 Etape 5

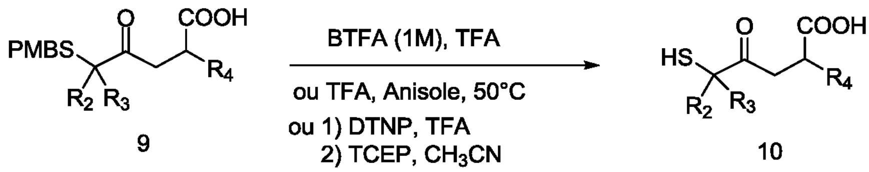


Méthode 1: Le composé **9** (0.53 mmol) de l'étape 7, est solubilisé dans 2.1mL de BTFA (boron tristrifluoroacetate) (1M) (préparé au préalable à partir de $BBr_3$ et de TFA) et est agité 1h à température ambiante. Les solvants sont évaporés sous pression réduite et le mélange est purifié par HPLC semi-préparative.

**10a** $R_2 = CH_2Ph$; $R_3 = H$; $R_4 = CH_2Ph$
ESI(+): $[M+H]^+$= 329
HPLC: ACE C18 $CH_3CN/H_2O$ (0.1% TFA) Gradient 60/90 en 30 min; Rt: 7.50 min
**10b** $R_2$ = iBu; $R_3$ = H; $R_4 = CH_2$(4-Br-Ph)
ESI(+): $[M+H]^+$= 372 et 374
HPLC: ACE C18 $CH_3CN/H_2O$ (0.1% TFA) Gradient 60/90 en 30 min; Rt: 8.60 min
**10c** $R_2 = CH_2$(4-Br-Ph); $R_3$ = H; $R_4 = CH_2$(4-Br-Ph)
ESI(+): $[M+H]^+$= 384
HPLC: ACE C18 $CH_3CN/H_2O$ (0.1% TFA) Gradient 60/90 en 30 min; Rt: 10.52 min
**10d** $R_2 = CH_2$(4-Br-Ph); $R_3$ = H; $R_4 = CH_2Ph$
ESI(+): $[M+H]^+$= 406 et 408
HPLC: ACE C18 $CH_3CN/H_2O$ (0.1% TFA) Gradient 60/90 en 30 min; Rt: 9.01 min
**10e** $R_2 = CH_2Ph$; $R_3$ = H; $R_4 = CH_2$(4-Br-Ph)
ESI(+): $[M+H]^+$= 406 et 408
HPLC: Kromasil C18 $CH_3CN/H_2O$ (0.1% TFA) 80/20; Rt: 6.23 min
**10f** $R_2 = CH_2$(4-Ph-Ph); $R_3$ = H; $R_4 = CH_2$(4-Br-Ph)
ESI(+): $[M+H]^+$= 482 et 484
HPLC: ACE C18 $CH_3CN/H_2O$ (0.1% TFA) Gradient 60/90 en 30 min; Rt: 11.4 min

Méthode 2: Le produit **9c** (2.03 mmol) est solubilisé dans 10mL de TFA en présence de 5 eq d'anisole (1.1 mL). Le mélange est chauffé 2.5h à 50°C. Les solvants sont évaporés sous pression réduite et le mélange est purifié par HPLC semi-préparative pour donner le composé **10b.**

**10b** $R_2$ = iBu; $R_3$ = H; $R_4 = CH_2$(4-Br-Ph)
ESI(+): $[M+H]^+$= 372 et 374
HPLC: ACE C18 $CH_3CN/H_2O$ (0.1% TFA) Gradient 60/90 en 30 min; Rt: 8.60 min

Méthode 3: A une solution, sous atmosphère inerte, du composé **9c** (0.447 mmol; 120mg) dans 4.5mL de TFA est ajouté le thioanisole (2.8eq; 0.936 mmol; 110μL) suivi par l'addition de 2,2'-dithiobis(5-nitropyridine) (3eq; 1.0 mmol; 417mg): la solution devient orangée. Le mélange est agité à température ambiante pendant 1h. Le mélange est concentré sous pression réduite à 30°C. Le disulfure formé est purifié par HPLC semi-préparative pour donner 146 mg de produit (Rdt: 62%).

Le disulfure précédent est solubilisé dans $CH_3CN$(720μL)/$H_20$(180μL). Le chlorydrate de la tris(2-carboxyethyl)phosphine (1.2eq; 96 mg) est alors ajouté et le mélange est agité 10 min à température ambiante. Les solvants sont évaporés sous pression réduite et le composé est purifié par HPLC semi-préparative pour donner **10b**

Ce schéma de synthèse permet de préserver la chiralité de la molécule si elle existe

**10b** $R_2$ = iBu; $R_3$ = H; $R_4 = CH_2$(4-Br-Ph)
ESI(+): $[M+H]^+$= 372 et 374
HPLC: ACE C18 $CH_3CN/H_2O$ (0.1% TFA) 60/90 en 30 min; Rt: 8.60 min
RMN ($CDCl_3$, 200MHz): 0.87 (3H, d); 0.89 (3H, d); 1.50-1.80 (3H, m); 2.7-3.5 (6H, m); 7.08 (2H, d); 7.43 (2H, d)

Les composés suivants sont obtenus selon un protocole identique.

**10g** $R_2 = CH_3$; $R_3 = CH_3$; $R_4 = CH_2$(4-Br-Ph)
ESI(+): $[M+Na]^+$= 366 et 368
HPLC: Atlantis T3 $CH_3CN/H_2O$ (0.1% TFA) 50/90 en 10 min; Rt: 6.51 min

RMN (DMSO d6, 200MHz): 1.45 (3H, s); 1.47 (3H, s); 2.7-3.2 (5H, m); 7.18 (2H, d); 7.50 (2H, d)
**10h** $R_2 = C_2H_5$; $R_3 = C_2H_5$; $R_4 = CH_2$(4-Br-Ph)
ESI(+): $[M+Na]^+$= 394 et 396
HPLC: Atlantis T3 $CH_3CN/H_2O$ (0.1% TFA) 70/90 en 10 min; Rt: 3.9 min
RMN (DMSO d6, 200MHz): 1.30 (6H, t); 1.75 (4H, q); 2.6-3.1 (5H, m); 7.0 (2H, d); 7.40 (2H, d)
**10i** $C(R_2)(R_3)$ = cyclopentyle; $R_4 = CH_2$(4-Br-Ph)
ESI(+): $[M+H]^+$= 370 et 372
HPLC: Atlantis T3 $CH_3CN/H_2O$ (0.1% TFA) 60/90 en 10 min; Rt: 5.0 min
RMN ($CDCl_3$, 200MHz): 1.60-2.25 (8H, m); 2.75-3.20 (5H, m); 7.10 (2H, d); 7.44 (2H, d)
**10j** $C(R_2)(R_3)$ = cyclohexyle; $R_4 = CH_2$(4-Br-Ph)
ESI(+): $[M+Na]^+$= 382 et 384
HPLC: Atlantis T3 $CH_3CN/H_2O$ (0.1% TFA) 70/90 en 10 min; Rt: 8.97 min
RMN ($CDCl_3$, 200MHz): 1.60-2.40 (10H, m); 2.75-3.20 (5H, m); 7.10 (2H, d); 7.44 (2H, d)
**10k** $C(R_2)(R_3)$ = indanyle; $R_4 = CH_2$(4-Br-Ph)
ESI(+): $[M+Na]^+$= 442 et 444
HPLC: Atlantis T3 $CH_3CN/H_2O$ (0.1% TFA) 70/90 en 10 min; Rt: 3.84 min
RMN ($CDCl_3$, 200MHz): 2.70-3.50 (5H, m); 3.60 (2H, d); 3.70 (2H, d); 7.10-7.6 (8H, m)
**10l** $C(R_2R_3)$ = cyclohexyle; $R_4 = CH_2$(4-Ph-Ph)
ESI(+): $[M+H]^+$= 383
HPLC: Atlantis T3 $CH_3CN/H_2O$ (0.1% TFA) 70/90 en 10 min; Rt: 5.35 min
RMN ($CDCl_3$, 200MHz): 1.60-2.40 (10H, m); 2.75-3.20 (5H, m); 7.20-7.60 (9H, m)
**10m** $C(R_2R_3)$ = cyclopentyle; $R_4 = CH_2$(4-Br-Ph)
ESI(+): $[M+H]^+$= 368 et 370
HPLC: Atlantis T3 $CH_3CN/H_2O$ (0.1% TFA) 60/40; Rt: 9.79 min
RMN ($CDCl_3$, 200MHz): 1.60-2.25 (8H, m); 2.75-3.20 (5H, m); 7.05-7.55(9H, m)
**10n** $C(R_2)(R_3)$ = indanyle; $R_4 = CH_2$(4-Ph-Ph)
ESI(+): $[M+H]^+$= 415
HPLC: Atlantis T3 $CH_3CN/H_2O$ (0.1% TFA) 70/90 en 10 min; Rt: 4.73 min
RMN ($CDCl_3$, 200MHz): 2.70-3.50 (5H, m); 3.60 (2H, d); 3.70 (2H, d); 7.10-7.8 (13H, m)

### 1.2 Synthèse des inhibiteurs mixte NEP-APN

#### 1.2.1 Synthèse du Boc-béta-aminothiol 11

Ces composés sont préparés en suivant le protocole décrit dans J.Med.Chem., 35,1992, 2473.

**11a** $R_1$: $CH_2CH_2SCH_3$; Solide blanc
HPLC: Atlantis T3, $CH_3CN$(0.1% TFA)/$H_2O$ (0.1% TFA) Gradient 50-90% 10 min, Rt = 6.45 min
RMN ($CDCl_3$, 200MHz): 1.45 (9H, s); 1.85 (2H, m); 2.12 (3H, s); 2.52 (2H, t); 2.75 (2H, dd); 3.90 (1H, t); 4.80 (1H, NH)
**11b** $R_1$: $CH_2CH_2CH_2CH_3$; Solide blanc
HPLC: ACE C18, $CH_3CN$(0.1% TFA)/$H_2O$ (0.1% TFA) 30%-70%, Rt = 13.53 min
RMN (DMSO d6, 200MHz): 0.95 (3H, t); 1.20-1.60 (6H, m); 1.40 (9H, s); 2.30 (2H, m); 3.40 (1H, t); 6.80 (1H, NH)
**11c** $R_1$: $CH_2CH_2OCH_3$; Solide blanc
HPLC: ACE C18, $CH_3CN$(0.1% TFA)/$H_2O$ (0.1% TFA) 50%-50%, Rt = 5.58 min
RMN (DMSO d6, 200MHz): 1.40 (9H, s); 1.60 (2H, m); 2.75-3.10 (2H, m); 3.20 (3H, s); 3.30 (2H, t); 3.60 (1H, m); 6.80 (1H, NH)
**11d** $R_1$: $CH_2CH_2OCH_2CH_3$; Solide blanc
HPLC: ACE C18, $CH_3CN$(0.1% TFA)/$H_2O$ (0.1% TFA) 40%-60%, Rt = 13.33 min
RMN (DMSO d6, 200MHz): 1.10 (3H, t); 1.40 (9H, s); 1.60 (2H, m); 2.60 (2H, m); 3.10-3.30 (2H, m); 3.30 (2H, q); 3.60 (1H, m); 6.80 (1H, NH)
**11e** $R_1$: $CH_2OCH_2CH_3$; Solide blanc
HPLC: ACE C18, $CH_3CN$(0.1% TFA)/$H_2O$ (0.1% TFA) 40%-60%, Rt = 14.00 min RMN (DMSO d6, 200MHz): 1.10 (3H, t); 1.40 (9H, s); 3.20-3.40 (4H, m); 3.30 (2H, q); 3.60 (1H, m); 6.70 (1H, NH)

#### 1.2.2 Synthèse du disulfure dissymétrique 12

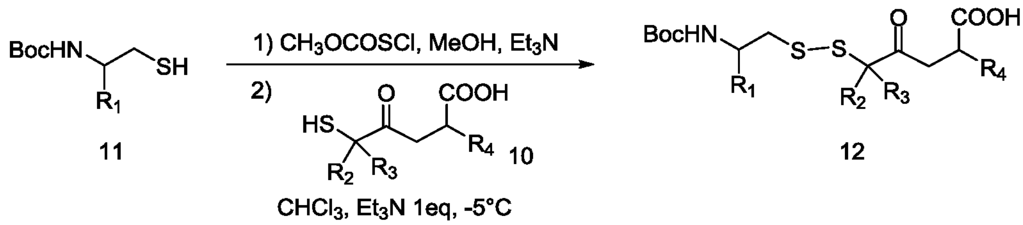


Le Boc-aminothiol thiol **11** (9.86 mmol, 2.5g) est mis en solution à 0°C, sous atmosphère inerte, dans 20 mL de MeOH dégazé. $Et_3N$ (2eq; 2.79 mL) est ajouté suivi par le chlorure de l'acide methoxycarbonylsulfonique (2eq, 1.78 mL) en solution dans 20mL de $CHCl_3$ dégazé. Le mélange est agité 15 min à 0°C puis 100mL de $CHCl_3$ sont ajoutés. La phase organique est lavée par ac. citrique 10% (2*100mL), $H_2O$ (100mL), NaCl sat. (100 mL), séchée sur $Na_2SO_4$ et concentrée sous pression réduite.

Le produit est purifié sur gel de silice

$R_1$: $CH_2CH_2SCH_3$; Solide blanc (Rdt: 40%)

HPLC: Atlantis T3, $CH_3CN$(0.1% TFA)/$H_2O$ (0.1% TFA) Gradient 70-90% en 10 min, Rt = 3.66 min

RMN ($CDCl_3$, 200MHz): 1.45 (9H, s); 1.85 (2H, m); 2.12 (3H, s); 2.52 (2H, t); 2.75 (2H, dd); 3.90 (1H, t); 3.90 (3H, s); 5.0 (1H, NH)

$R_1$: $CH_2CH_2CH_2CH_3$; Solide blanc (Rdt: 41%)

HPLC: Atlantis T3, $CH_3CN$(0.1% TFA)/$H_2O$ (0.1% TFA) Gradient 70-90% en 10 min, Rt = 5.57 min

RMN ($CDCl_3$, 200MHz): 0.90 (3H, t); 1.34 (4H, m); 1.45 (9H, s); 1.62 (2H, m); 2.99 (2H, d); 3.78 (1H, m); 3.90 (3H, s); 4.77 (1H, NH)

A une solution du composé précédent (0.754 mmol, 1eq) dans 8mL de $CHCl_3$ dégazé, sous atmosphère inerte, est ajouté le composé **10.** Le mélange est refroidit à -10°C et $Et_3N$ dégazée (0.754 mmol, 105μL, 1eq) est ajoutée. Le mélange est agité 30 min à -10°c puis est dilué par 10mL de $CH_2Cl_2$. La phase organique est lavée par ac.citrique 10% (5mL), NaCl sat. (2*10mL), séchée sur $Na_2SO_4$ pour donner un produit brut, qui est purifié par HPLC semipréparative pour donner le composé **12.**

**12a-b** $R_1$: $CH_2CH_2SCH_3$; $R_2$: iBu; $R_3$: H; $R_4$: $CH_2$(4-Br-Ph) (Rdt: 64%)
ESI(+): $[M+Na]^+$= 644 et 646
**12b-b** $R_1$: $CH_2CH_2CH_2CH_3$; $R_2$: iBu; $R_3$: H; $R_4$: $CH_2$(4-Br-Ph) (Rdt: 87%)
ESI(+): $[M+Na]^+$= 626 et 628
**12b-l** $R_1$: $CH_2CH_2CH_2CH_3$; $C(R_2R_3)$: cyclohexyle; $R_4$: $CH_2$(4-Ph-Ph) (Rdt: 85%)
ESI(+): $[M+Na]^+$= 637
**12b-j** $R_1$: $CH_2CH_2CH_2CH_3$; $C(R_2R_3)$: yclohexyle; $R_4$: $CH_2$(4-Br-Ph) (Rdt: 90%)
ESI(+): $[M+Na]^+$= 638 et 640
**12a-l** $R_1$: $CH_2CH_2SCH_3$; $C(R_2\ R_3)$: yclohexyle; $R_4$: $CH_2$(Ph-Ph) (Rdt: 90%)
ESI(+): $[M+H]^+$= 632

### 1.2.3 Préparation des composés 1

Procédé 1

Partie 1: synthèse des esters

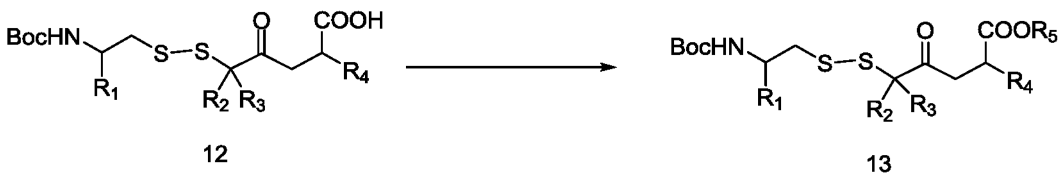

**Composé 13a-b-1: 1-(ethoxycarbonyloxy)ethyl 2-(4-bromobenzyl)-5-(((S)-2-(tert-butoxycarbonylamino)-4-(methylthio)butyl)disulfanyl)-7-methyl-4-oxooctanoate**

Le composé **12a-b** (640mg; 1.03 mmol) et $Et_3N$ (730µL, 5 eq) sont dissous dans 10 mL d'AcOEt. Le mélange est agité 15 min à température ambiante. L'éthyl-1-chloroéthylcarbonate (préparé selon Barcelo et al. Synthesis, 1986, 627) (800µL; 5eq) et NaI (800 mg, 5 eq) sont ajoutés. Le mélange est porté 3h à reflux. Le mélange est dilué par 10 mL $H_2O$ et 20 mL AcOEt. La phase aqueuse est extraite par 3x30 mL AcOEt. La phase organique est lavée par acide citrique 10% (2*15 mL), $NaHCO_3$ 10% (2*15 mL), NaCl sat., séchée sur $Na_2SO_4$ et évaporée sous pression réduite pour donner un produit brut. Le mélange est purifié par HPLC semipréparative pour donner 80mg d'une huile jaunâtre.

**13a-b-1** $R_1$: $CH_2CH_2SCH_3$; $R_2$: iBu; $R_3$: H; $R_4$: $CH_2$(4-Br-Ph); $R_5$: $CH(CH_3)OCOOC_2H_5$ (Rdt 10.5%)
HPLC: Atlantis T3, $CH_3CN$(0.1% TFA)/$H_2O$ (0.1% TFA) Gradient 70-90% en 10 min, Rt = 12.85 min
ESI(+) : $[M+Na]^+$= 760 et 762

**Composé 13b-b-1: ethyl 2-(4-bromobenzyl)-5-(((S)-2-(tert-butoxycarbonylamino)hexyl) disulfanyl)-7-methyl-4-oxooctanoate**

Le composé **12b-b** (395mg; 0.653 mmol) est dissous à 4°C, sous atmosphère inerte, dans 4.3 mL de $CH_2Cl_2$. EDCI-HCl (138mg; 0.718 mmol; 1.1 eq) est ajouté, suivi par DMAP (88mg; 0.718 mmol; 1.1 eq) et EtOH (0.784 mmol; 1.2 eq). Le mélange est agité 4h à température ambiante. Le mélange est dilué par 10 mL acide citrique 10% et 20 mL $CH_2Cl_2$. La phase organique est lavée par acide citrique 10% (2*10 mL), $NaHCO_3$ 10% (2*10 mL), NaCl sat., séchée sur $Na_2SO_4$ et évaporée sous pression réduite pour donner un produit brut. Le mélange est purifié par HPLC semipréparative pour donner une huile jaunâtre.

**13b-b-1** $R_1$: $CH_2CH_2CH_2CH_3$; $R_2$: iBu; $R_3$: H; $R_4$: $CH_2$(4-Br-Ph); $R_5$: $C_2H_5$ (Rdt 60%) HPLC: Atlantis T3, $CH_3CN$(0.1% TFA)/$H_2O$ (0.1% TFA) Gradient 80-90% en 10 min, Rt = 12.20-12.65 min

<u>Partie 2: déprotection de l'amine</u>

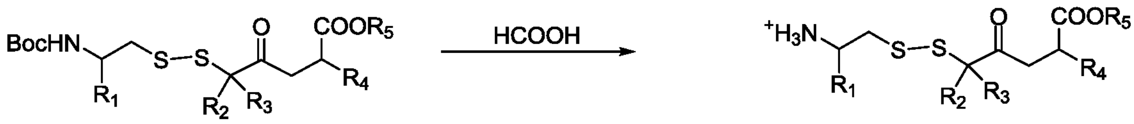


13 1

**Composé la-b-1: 1-(ethoxycarbonyloxy)ethyl 5-(((S)-2-amino-4-(methylthio)butyl) disulfanyl)-2-(4-bromobenzyl)-7-methyl-4-oxooctanoate**

Le composé **13a-b-1** (130mg; 0.176 mmol) est agité dans 2mL HCOOH pendant 1h. Le mélange est évaporé sous pression réduite pour donner un produit brut qui est purifié par HPLC semipréparative pour donner 13 mg du composé **1a-b-1.**

**1a-b-1** $R_1$: $CH_2CH_2SCH_3$; $R_2$: iBu; $R_3$: H; $R_4$: $CH_2$(4-Br-Ph); $R_5$: $CH(CH_3)OCOOC_2H_5$ (Rdt 11%)
HPLC: Atlantis T3, $CH_3CN$(0.1% TFA)/$H_2O$ (0.1% TFA) Gradient 10-90% en 15 min, Rt = 14.50 min
ESI(+) : $[M+H]^+$= 638 et 640

**Composé 1b-b-1: ethyl 5-(((S)-2-aminohexyl)disulfanyl)-2-(4-bromobenzyl)-7-methyl-4-oxooctanoate**

Le composé **13b-b-1** (141mg; 0.223 mmol) est agité dans 2mL HCOOH pendant 1h. Le mélange est évaporé sous pression réduite pour donner un produit brut qui est purifié par HPLC semipréparative pour donner 92 mg du composé **1b-b-1.**

**1b-b-1** $R_1$: $CH_2CH_2CH_2CH_3$; $R_2$: iBu; $R_3$: H; $R_4$: $CH_2$(4-Br-Ph); $R_5$: $C_2H_5$ (Rdt 64%) HPLC: Atlantis T3, $CH_3CN$(0.1% TFA)/$H_2O$ (0.1% TFA) Gradient 50-90% en 10 min, Rt = 7.79 min
ESI(+) : $[M+H]^+$= 532 et 534

**Composé 1f-b-1: ethyl 5-((2-amino-4-(methylsulfinyl)butyl)disulfanyl)-2-(4-bromobenzyl)-7-methyl-4-oxooctanoate**

**1f-b-1** $R_1$: $CH_2CH_2SOCH_3$; $R_2$: iBu; $R_3$: H; $R_4$: $CH_2$(4-Br-Ph); $R_5$: $C_2H_5$ (Rdt 47.9%) HPLC: Atlantis T3, $CH_3CN$(0.1% TFA)/$H_2O$ (0.1% TFA) Gradient 10-90% en 15 min, Rt = 12.45 min
ESI(+) : $[M+H]^+$= 566 et 568

Procédé 2

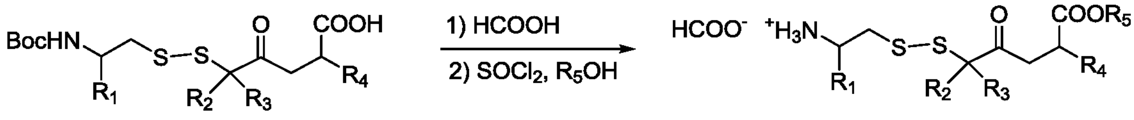


12 1

Le composé **12** (0.323 mmol) est agité dans 4mL HCOOH pendant 1h. Le mélange est évaporé sous pression réduite pour donner un produit brut qui est purifié par HPLC semipréparative pour donner l'acide.
$R_1$: $CH_2CH_2SCH_3$; $R_2$: iBu; $R_3$: H; $R_4$: $CH_2$(4-Br-Ph) (Rdt 69%)
HPLC: ACE C18, $CH_3CN$(0.1% TFA)/$H_2O$ (0.1% TFA) 60/40%, Rt = 8.0 min
ESI(+) : $[M+H]^+$= 522 et 524
$R_1$: $CH_2CH_2CH_2CH_3$; $C(R_2)(R_3)$: cyclohexyle; $R_4$: $CH_2$(4-Ph-Ph) (Rdt 60%)
HPLC: Luna T3, $CH_3CN$(0.1% TFA)/$H_2O$ (0.1% TFA) 60/40%, Rt = 2.83 min
ESI(+) : $[M+H]^+$= 514
$R_1$: $CH_2CH_2CH_2CH_3$; $C(R_2)(R_3)$: cyclohexyle; $R_4$: $CH_2$(4-Br-Ph) (Rdt 69%)
HPLC: ACE C18, $CH_3CN$(0.1% TFA)/$H_2O$ (0.1% TFA) 60/40%, Rt = 8.0 min
ESI(+) : $[M+H]^+$= 516 et 518
$R_1$: $CH_2CH_2SCH_3$; $C(R_2)(R_3)$: cyclohexyle; $R_4$: $CH_2$(4-Ph-Ph) (Rdt 70%)
HPLC: Luna C18, $CH_3CN$(0.1% TFA)/$H_2O$ (0.1% TFA) 50/50%, Rt = 5.43 min
ESI(+) : $[M+H]^+$= 532

$SOCl_2$ (30μL, 6eq) est ajouté à une suspension de l'acide précédent (0.068 mmol) à 0°C, sous atmosphère inerte, dans 400μL de EtOH ($R_5$=Et) anhydre. La solution devient limpide. Le mélange est agité la nuit à température ambiante. Le mélange est évaporé sous pression réduite pour donner un produit brut qui est purifié par HPLC semipréparative pour donner 35 mg du produit attendu.

**1a-b-2** $R_1$: $CH_2CH_2SCH_3$; $R_2$: iBu; $R_3$: H; $R_4$: $CH_2$(4-Br-Ph); $R_5$: $C_2H_5$ (Rdt 82%)
HPLC: ACE C18, $CH_3CN$(0.1% TFA)/$H_2O$ (0.1% TFA) Gradient 50/90% en 30 min, Rt = 11.98 min
ESI(+) : $[M+H]^+$= 550 et 552
**1b-l-1** $R_1$: $CH_2CH_2CH_2CH_3$; $C(R_2)(R_3)$: $C_5H_{10}$; $R_4$: $CH_2$(4-Ph-Ph); $R_5$: $C_2H_5$ (Rdt 80%) HPLC: Atlantis T3, $CH_3CN$(0.1% TFA)/$H_2O$ (0.1% TFA) Gradient 70/90% en 10 min, Rt = 4.0 min
ESI(+) : $[M+H]^+$= 542
**1b-j-1** $R_1$: $CH_2CH_2CH_2CH_3$; $C(R_2)(R_3)$: cyclohexyle; $R_4$: $CH_2$(4-Br-Ph); $R_5$: $C_2H_5$ (Rdt 85%) HPLC: Atlantis T3, $CH_3CN$(0.1% TFA)/$H_2O$ (0.1% TFA) Gradient 70/90% en 10 min, Rt = 3.28 min
ESI(+) : $[M+H]^+$= 546 et 548
**1a-l-1** $R_1$: $CH_2CH_2SCH_3$; $C(R_2)(R_3)$: cyclohexyle; $R_4$: $CH_2$(4-Ph-Ph); $R_5$: $C_2H_5$ (Rdt 86%) HPLC: Atlantis T3, $CH_3CN$(0.1% TFA)/$H_2O$ (0.1% TFA) Gradient 50/90% en 10 min, Rt = 8.42 min
ESI(+) : $[M+H]^+$= 560
**1b-l-2** $R_1$: $CH_2CH_2CH_2CH_3$; $C(R_2)(R_3)$: cyclohexyle; $R_4$: $CH_2$(4-Ph-Ph); $R_5$: $CH_2Ph$ (Rdt 71%)
HPLC: Atlantis T3, $CH_3CN$(0.1% TFA)/$H_2O$ (0.1% TFA) Gradient 70/90% en 10 min, Rt = 5.71 min
ESI(+) : $[M+H]^+$= 604

Procédé 3

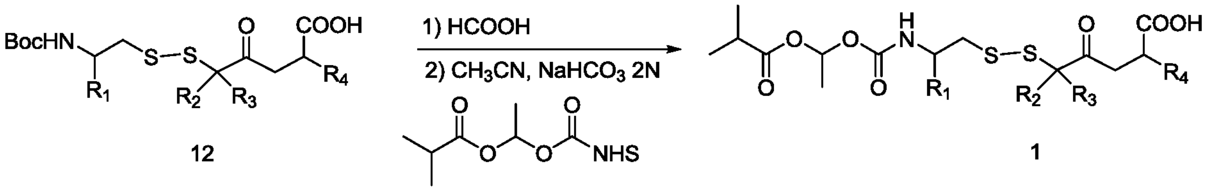


12 1

**Composé 1b-I-3: Acide 2-(biphenyl-4-ylmethyl)-4-(1-((2-((1-(isobutyryloxy)ethoxy) carbonylamino)(S)-hexyl)disulfanyl)cyclohexyl)-4-oxobutanoique**

Le composé **12b-I** (590mg; 0.961 mmol) est agité dans 10mL HCOOH pendant 1h. Le mélange est évaporé sous pression réduite pour donner un produit brut qui est purifié par HPLC semipréparative pour donner les 2 diastéréoisomères.
$R_1$: $CH_2CH_2CH_2CH_3$; $C(R_2R_3)$: cyclohexyle; $R_4$: $CH_2$(4-Ph-Ph) (Rdt 89%)
HPLC Dia 1: Luna C18, $CH_3CN$(0.1% TFA)/$H_2O$ (0.1% TFA) 55/35%, Rt = 3.90 min
HPLC Dia 2: Luna C18, $CH_3CN$(0.1% TFA)/$H_2O$ (0.1% TFA) 55/35%, Rt = 4.10 min
ESI(+) : $[M+H]^+$= 522.2

Le composé précédent (155mg, 0246 mmol) est solubilisé dans 2mL de $CH_3CN$ anhydre. 370µL de $NaHCO_3$ 2N sont ajoutés, suivi par 1mL $H_2O$. Le mélange est agité 10 min à TA et 1-((2, 5-dioxopyrrolidin-1-yloxy)carbonyloxy)ethyl isobutyrate (90mg ; .329 mmol ; 1eq) (Cundy et al. (2004) J. Pharm. Exp. Therap., 311, 315-323) dans $CH_3CN$ (1mL) sont ajoutés. Le mélange est agité 30 min à 60°C. Le solvant est évaporé sous pression réduite. Le produit est repris dans AcOEt, HCl 1N. La phase organique est lavée, séchée sur $Na_2SO_4$, concentrée sous pression réduite pour donner un produit brut qui est purifié par HPLC semipréparative pour donner le produit attendu.

**1b-I-3** dia 1 $R_1$: $CH_2CH_2CH_2CH_3$; $C(R_2)(R_3)$: cyclohexyle; $R_4$: $CH_2$(4-Ph-Ph) (Rdt 85%) HPLC: Atlantis T3, $CH_3CN$(0.1% TFA)/$H_2O$ (0.1% TFA) Gradient 70/90% en 15 min, Rt = 10.50 min et 10.80 min
ESI(+) : $[M+H]^+$= 672.1
**1b-I-3** dia 2 $R_1$: $CH_2CH_2CH_2CH_3$; $C(R_2)(R_3)$: cyclohexyle; $R_4$: $CH_2$(4-Ph-Ph) (Rdt 85%) HPLC: Atlantis T3, $CH_3CN$(0.1% TFA)/$H_2O$ (0.1% TFA) Gradient 70/90% en 15 min, Rt = 10.80 min et 11.10 min
ESI(+) : $[M+H]^+$= 672.1

## 2. Mesure des pouvoirs inhibiteurs

Les dosages sont effectués en plaque 96 puits en présence d'un substrat fluorigénique spécifique. La lecture de la fluorescence émise est faite dans un lecteur de plaque Berthold Twinkle LS970B. On réalise ensuite une courbe d'inhibition en fonction de la concentration en inhibiteur à l'aide du logiciel GraphPad, puis on détermine le Ki à partir de la formule de Cheng Prusoff : $Ki=IC_{50}/(1 +(S/Km))$.

Les pouvoirs inhibiteurs sur les 2 enzymes cibles sont déterminés :

- A partir des pro-drogues **1** après coupure *in situ* des protections N et/ou C terminal et du pont disulfure
- Les valeurs précédentes sont contrôlées à partir des inhibiteurs sélectifs intermédiaires dans la synthèse des pro-drogues c'est-à-dire les composés **10** pour la Néprilysine et **11,** après déprotection de la fonction amine pour l'APN.

*Inhibition de l'activité néprilysine (NEP)*

La néprilysine, purifiée à partir de rein de lapin (Aubry M. et al. 1987, Biochem. Cell. Biol. 65, 398-404), est utilisée à 200ng/mL final dans le tampon Tris 50mM pH 7.4. Le substrat, Dansyl-Gly-($NO_2$)Phe-β-Ala (Goudreau N.et al. (1994) Anal. Biochem., 219, 87-95) (Km=37 µM), est dissous dans l'éthanol et est utilisé à 20µM final. Des concentrations croissantes (de $10^{-10}$ à $10^{-3}$ M) d'inhibiteurs sont pré-incubées pendant 15 min à 37°C avec la NEP-1 dans le tampon Tris 50mM, pH 7.4. Le substrat est ensuite ajouté et l'incubation est poursuivie pendant 60 min. La réaction est arrêtée en mettant la plaque dans la glace pendant 10 min. La lecture de la fluorescence émise est mesurée dans un fluorimètre à λex= 355nm, λem= 535nm. Les résultats sont présentés dans les tableaux suivants.

| **Composé** | | **$R_2$** | **$R_3$** | **$R_4$** | **$R_5$** | **NEP-1 Ki (nM)** |
|---|---|---|---|---|---|---|
| **10 a** | Dia 1 +Dia 2 | $CH_2Ph$ | H | $CH_2Ph$ | OH | 73 ± 8 |

(suite)

| Composé | | $R_2$ | $R_3$ | $R_4$ | $R_5$ | NEP-1 Ki (nM) |
|---|---|---|---|---|---|---|
| **10 a** | Dia 3+Dia 4 | $CH_2Ph$ | H | $CH_2Ph$ | OH | 1500 ± 200 |
| **10 b** | 4 Dia | iBu | H | $CH_2$(4-Br-Ph) | OH | 20 ± 5 |
| **10 c** | Dia 1 +Dia 2 | $CH_2$(4-Br-Ph) | H | $CH_2$(4-Br-Ph) | OH | 310 ± 25 |
| **10 c** | Dia 3+ Dia 4 | $CH_2$(4-Br-Ph) | H | $CH_2$(4-Br-Ph) | OH | 130 ± 40 |
| **10 d** | Dia 1 +Dia 2 | $CH_2$(4-Br-Ph) | H | $CH_2Ph$ | OH | 2300 ± 300 |
| **10 d** | Dia 3+ Dia 4 | $CH_2$(4-Br-Ph) | H | $CH_2Ph$ | OH | 1230 ± 300 |
| **10 e** | 4 Dia | $CH_2Ph$ | H | $CH_2$(4-Br-Ph) | OH | 50 ± 8 |
| **10 f** | 4 Dia | $CH_2$(4-Ph-Ph) | H | $CH_2$(4-Br-Ph) | OH | 250 ± 50 |
| **10 g** | 2 stéréo | $CH_3$ | $CH_3$ | $CH_2$(4-Br-Ph) | OH | 470 ± 40 |
| **10 h** | 2 stéréo | $C_2H_5$ | $C_2H_5$ | $CH_2$(4-Br-Ph) | OH | 86 ± 9 |
| **10 i** | 2 stéréo | 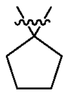 | | $CH_2$(4-Br-Ph) | OH | 52 ± 4 |
| **10 j** | 2 stéréo | 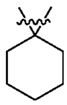 | | $CH_2$(4-Br-Ph) | OH | 43 ± 3 |
| **10 k** | 2 stéréo | 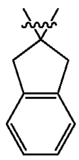 | | $CH_2$(4-Br-Ph) | OH | 36 ± 3 |
| **10 l** | 2 stéréo | 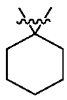 | | $CH_2$(4-Ph-Ph) | OH | 9 ± 1 |
| **10 m** | 2 stéréo | 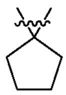 | | $CH_2$(4-Ph-Ph) | OH | 29 ± 5 |
| **10 n** | 2 stéréo | 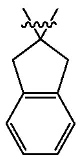 | | $CH_2$(4-Ph-Ph) | OH | 26 ± 1 |

| Composé | | $R_2$ | $R_3$ | $R_4$ | $R_5$ | NEP-1 Ki (nM) |
|---|---|---|---|---|---|---|
| **10 b** | Dia | (R ou S)- iBu | H | (R)-$CH_2$(4-Br-Ph) | OH | 15 ± 1 |
| **10 b** | Dia 2 | (R ou S)-iBu | H | (R)-$CH_2$(4-Br-Ph) | OH | 12 ± 0.5 |
| **10 b** | Dia 3 | (R ou S)-iBu | H | (S)-$CH_2$(4-Br-Ph) | OH | 6 ± 0.5 |
| **10 b** | Dia 4 | (R ou S)- iBu | H | (S)-$CH_2$(4-Br-Ph) | OH | 5 ± 0.4 |
| **10 l** | Stéréo 1 | 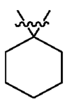 | | (R)-$CH_2$(4-Ph-Ph) | OH | 4.2 ± 0.1 |

(suite)

| Composé | | $R_2$ | $R_3$ | $R_4$ | $R_5$ | NEP-1 Ki (nM) |
|---|---|---|---|---|---|---|
| **10 l** | Stéréo 2 | 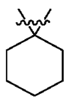 | | (S)-$CH_2$(4-Ph-Ph) | OH | 36 ± 0.7 |

*Inhibition de l'activité APN*

La mesure de l'inhibition de l'aminopeptidase N (APN) est effectuée par utilisation du substrat L-Ala↓β-NA (50μM, Sigma Aldrich. Les pouvoirs inhibiteurs sont déterminés en utilisant de l'enzyme humaine recombinante (rh) (50 ng/mL; R&D System). Des concentrations croissantes (de $10^{-10}$ à $10^{-3}$ M) d'inhibiteurs sont pré-incubées pendant 30 min à 37°C avec l'APN-rh dans le tampon Tris 50mM, pH 7.4. Le substrat est ensuite ajouté et l'incubation est poursuivie pendant 30 min à 37°C. La réaction est arrêtée en mettant la plaque dans la glace pendant 10 min. La lecture de la fluorescence émise est mesurée dans un fluorimètre à λex= 340nm, λem= 405nm. Les résultats sont présentés dans le tableau suivant.

$CF_3COO^-$ 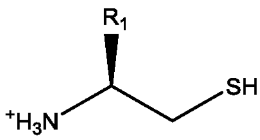

| Composé | $R_1$ | Ki APN (nM) |
|---|---|---|
| **a** | $CH_2CH_2SCH_3$ | 11 ± 2 |
| **b** | $CH_2CH_2CH_2CH_3$ | 13 ± 2 |
| **c** | $CH_2CH_2OCH_3$ | 35 ± 2 |
| **d** | $CH_2CH_2OCH_2CH_3$ | 47 ± 8 |
| **e** | $CH_2OCH_2CH_3$ | 55 ± 10 |

**3. Pharmacologie: test de la plaque chaude**

Le réflexe de saut chez une souris placée sur une plaque chauffée à 52°C est mesuré par le temps mis par l'animal à sauter pour échapper à la douleur (latence du saut) (Eddy, N.B. et al. J. Pharm. Exp. Therap., 1953, 107, 385-389).

Le composé de formule **(1a-b-1 Dia 3)** ou **(1b-l-1 Dia 1)** est injecté par voie intraveineuse chez des souris OF1 mâles (23-26g) (10 mg/kg) après dissolution dans un mélange éthanol/tween 80/eau (1/1/8).

Volume d'injection: 10 mL/kg

**TFA**

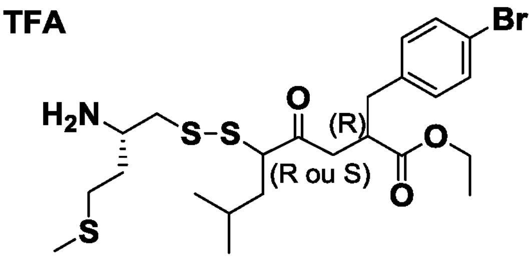

**(1a-b-1 Dia 3)**

**TFA**

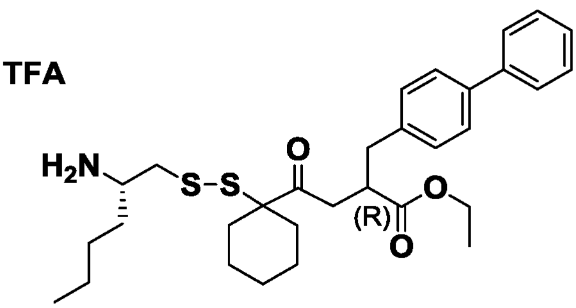

**(1b-l-1 Dia 1)**

Les temps de latence des sauts sont mesurés 10 min après les injections par voie intraveineuse.
Les résultats sont exprimés en pourcentage d'analgésie en utilisant l'équation suivante:

$$\% \text{ analgésie} = \frac{(\text{temps de latence mesuré} - \text{temps de latence contrôle})}{(\text{temps de latence maximum} - \text{temps de latence contrôle})}$$

Temps de latence maximum = 240 secondes.
Les résultats sont exprimés en terme de moyenne ± s.e.m. Les résultats sont représentés sur la figure 1.

## Revendications

1. Composé de formule générale **(1)**

**(1)** **R**-NH-CH(**$R_1$**)-$CH_2$-S-S-C(**$R_2$**)(**$R_3$**)-$COCH_2$-CH(**$R_4$**)-CO**$R_5$**

dans laquelle :

a) **R** représente :

- un hydrogène ;
- un groupement alkoxyalkylcarbonyl R'C(O)OCH(R")OC(O)- où R' et R" représentent, indépendamment l'un de l'autre, un groupement alkyle contenant de 1 à 6 atomes de carbone ;

b) **$R_1$** représente un groupement alkyle, linéaire ou ramifié, de 1 à 6 atomes de carbone, substitué ou non, par un groupement -OR'", -SOR'" ou -SR'", avec R'" représentant un groupement alkyle de 1 à 6 atomes de carbone, substitué ou non, par un ou plusieurs atomes d'halogènes ;
c) **$R_2$** représente :

- un groupement alkyle, linéaire ou ramifié, de 1 à 6 atomes de carbone, substitué ou non, par:

  - un groupement -$OR_6$, -$SR_6$ ou -$SOR_6$, avec $R_6$ représentant un hydrogène, un groupement alkyle linéaire ou ramifié de 1 à 4 carbones, un groupement phényle ou benzyle ;
  - un groupement -$CO_2R_7$, avec $R_7$ représentant un hydrogène, un groupement alkyle, linéaire ou ramifié, comprenant de 2 à 4 atomes de carbone ou un groupement benzyle ;
  - un groupement -$NR_8R_9$, avec $R_8$ et $R_9$ représentant indépendamment l'un de l'autre un hydrogène, un groupement alkyle, linéaire ou ramifié, de 1 à 4 atomes de carbone, un groupement phényle ou benzyle, ou avec -$NR_8R_9$ pris ensemble représentant un hétérocycle saturé à 5 ou 6 chaînons comportant un ou plusieurs hétéroatomes choisis parmi N ou O, de préférence représentant une morpholine ou une pipéridine; ou
  - un groupement carboxamide -$CONR_8R_9$, avec -$NR_8R_9$ tel que défini ci-dessus ;
  - un groupement phényle, substitué, ou non, par un ou plusieurs halogènes pris parmi le fluor ou le brome, un groupement alkoxy -$OR_6$, avec $R_6$ ayant la même définition que ci-dessus ou par un groupement phényle;
  - un hétérocycle aromatique à 5 ou 6 chaînons comprenant 1 ou 2 hétéroatomes choisi(s) parmi l'oxygène, l'azote et le soufre;
  - un composé cyclique saturé à 5 ou 6 chaînons ou hétérocyclique saturé à 5 ou 6 chaînons comprenant 1 ou 2 hétéroatomes choisis parmi l'oxygène, l'azote et le soufre ;

- un composé cyclique saturé à 5 ou 6 chaînons, ou hétérocyclique saturé à 5 ou 6 chaînons comprenant 1 ou 2 hétéroatomes choisis parmi l'oxygène, l'azote et le soufre ; ou
- un groupement phényle substitué ou non par un ou plusieurs halogènes pris parmi le fluor ou le brome, ou par un groupement -$OR_5$ avec $R_5$ ayant la même définition que ci-dessus ;

et **$R_3$** représente un hydrogène ; ou
**$R_2$** et **$R_3$** sont identiques et représentent un groupement alkyle, linéaire ou ramifié de 1 à 6 atomes de carbone ; ou

**$-C(R_2)(R_3)-$** pris ensemble représente :

- un composé cyclique saturé à 5 chaînons accolé, ou non, à un cycle aromatique, de préférence conduisant au cycle indanyle;
- un composé cyclique saturé à 6 chaînons ;
- un composé hétérocyclique saturé à 6 chaînons comprenant 1 hétéroatome, en position 4, choisi parmi l'oxygène, l'azote et le soufre, dans lequel lorsque l'hétéroatome est l'azote, l'azote est substitué, ou non, par un groupement alkyle de 1 à 6 atomes de carbone, un groupement phényle, benzyle ou un groupement alcanoyle;

d) **$R_4$** représente :

- un groupement alkyle, linéaire ou ramifié, de 1 à 6 atomes de carbone, substitué ou non, par:

  - un groupement $-OR_6$, $-SR_6$ ou $-SOR_6$, avec $R_6$ représentant un hydrogène, un groupement alkyle linéaire ou ramifié de 1 à 4 carbones, un groupement phényle ou benzyle ;
  - un groupement $-CO_2R_7$, avec $R_7$ représentant un hydrogène, un groupement alkyle, linéaire ou ramifié, comprenant de 2 à 4 atomes de carbone, un groupement benzyle ;
  - un groupement $-NR_8R_9$, avec $R_8$ et $R_9$ représentant indépendamment l'un de l'autre un hydrogène, un groupement alkyle, linéaire ou ramifié, de 1 à 4 atomes de carbone, un groupement phényle ou benzyle, ou avec $-NR_8R_9$ pris ensemble représentant un hétérocycle saturé à 5 ou 6 chaînons comportant un ou plusieurs hétéroatomes choisis parmi N ou O, de préférence représentant une morpholine ou une pipéridine;
  - un groupement carboxamide $-CONR_8R_9$, avec $-NR_8R_9$ tel que défini ci-dessus ;
  - un groupement phényle substitué, ou non, par :

    - un ou plusieurs halogènes pris parmi le fluor ou le brome ;
    - un groupement $-OR_6$, $R_6$ ayant la même définition que ci-dessus ;
    - un groupement phényle ou thiényle ;

  - un hétérocycle aromatique à 5 ou 6 chaînons comprenant 1 ou 2 hétéroatomes choisi(s) parmi l'oxygène, l'azote et le soufre ; ou
  - un composé cyclique saturé à 5 ou 6 chaînons ou hétérocyclique saturé à 5 ou 6 chaînons comprenant 1 ou 2 hétéroatomes choisis parmi l'oxygène, l'azote et le soufre ;

- un groupement phényle, substitué ou non, par :

  - un ou plusieurs halogènes notamment le fluor ou le brome ;
  - un groupement $-OR_6$, avec $R_6$, ayant la même définition que ci-dessus ;
  - un phényle ;
  - un hétérocycle aromatique à 5 ou 6 chaînons comprenant 1 ou 2 hétéroatomes choisis parmi l'oxygène, l'azote et le soufre ;

e) **$R_5$** représente :

- un groupement hydroxyle ;
- un groupement $-NR_8R_9$, avec $R_8$ et $R_9$ représentant indépendamment l'un de l'autre un hydrogène, un groupement alkyle, linéaire ou ramifié, de 1 à 4 atomes de carbone, un groupement phényle ou benzyle, ou avec $-NR_8R_9$ pris ensemble représentant un hétérocycle à 5 ou 6 chaînons, comportant un ou plusieurs hétéroatomes choisis parmi N ou O, de préférence représentant une morpholine ou une pipéridine ;
- un groupement alkoxy $-OR_{10}$, avec $R_{10}$ représentant :

  - un groupement alkyle, linéaire ou ramifié, comportant de 2 à 6 atomes de carbone ;
  - un groupement benzyle ;
  - un groupement $-CHR_{11}-COOR_{12}$, $-CHR_{11}-O-C(=O)R_{12}$, $-CHR_{11}$-ou $-C(=O)-OR_{12}$ dans lesquels $R_{11}$ et $R_{12}$ représentent, indépendamment l'un de l'autre, un groupement alkyle, linéaire ou ramifié, de 1 à 6 atomes de carbone.

2. Composé selon la revendication 1 dans lequel $R_2$ représente un groupement alkyle linéaire ou ramifié de 1 à 6 atomes de carbone ou un groupement alkyle linéaire ou ramifié de 1 à 6 atomes de carbone substitué par:

- un groupement phényle ;
- un groupement phényle substitué par un ou plusieurs halogènes pris parmi le fluor ou le brome ;
- un hétérocycle aromatique à 5 ou 6 chaînons comprenant 1 ou 2 hétéroatomes choisi(s) parmi l'oxygène, l'azote et le soufre ;
- un composé cyclique saturé à 5 ou 6 chaînons ou hétérocyclique saturé à 5 ou 6 chaînons comprenant 1 ou 2 hétéroatomes choisis parmi l'oxygène, l'azote et le soufre ;

et $R_3$ représente un hydrogène, ou
$R_2$ et $R_3$ sont identiques et représentent un groupement alkyle linéaire ou ramifié de 1 à 6 atomes de carbone, ou
**-C(R₂)(R₃)-** pris ensemble représente :

- un composé cyclique saturé à 5 chaînons,
- un composé cyclique saturé à 5 chaînons accolé à un cycle aromatique ;
- un composé cyclique saturé à 6 chaînons ; ou
- un composé hétérocyclique saturé à 6 chaînons comprenant 1 hétéroatome, en position 4, choisi parmi l'oxygène, l'azote et le soufre.

3. Composé selon la revendication 1 ou 2 dans lequel $R_4$ représente un groupement alkyle linéaire ou ramifié de 1 à 6 atomes de carbones substitué par:

- un groupement phényle; ou
- un groupement phényle substitué par:

  - un ou plusieurs halogènes pris parmis le fluor ou le brome;
  - un groupement phényle ou thiényle.

4. Composé selon l'une des revendications précédentes dans lequel $R_5$ représente un hydroxyle.

5. Composé selon l'une des revendications précédentes dans lequel:

a) $R_1$ est choisi parmi $-CH_2CH_2SCH_3$, $-CH_2CH_2SOCH_3$, $-CH_2CH_2CH_2CH_3$;
b) $R_2$ représente un groupement alkyle linéaire ou ramifiée de 1 à 6 atomes de carbone ou un groupement alkyle linéaire ou ramifiée de 1 à 6 atomes de carbone substitué par:

- un groupement phényle ;
- un groupement phényle substitué par un ou plusieurs halogènes pris parmi le fluor ou le brome ;
- un hétérocycle aromatique à 5 ou 6 chaînons comprenant 1 ou 2 hétéroatomes choisi(s) parmi l'oxygène, l'azote et le soufre ;
- un composé cyclique saturé à 5 ou 6 chaînons ou hétérocyclique saturé à 5 ou 6 chaînons comprenant 1 ou 2 hétéroatomes choisis parmi l'oxygène, l'azote et le soufre ;

et $R_3$ représente un hydrogène, ou
$R_2$ et $R_3$ sont identiques et représentent un groupement alkyle linéaire ou ramifié de 1 à 6 atomes de carbone ; ou
**-C(R₂)(R₃)-** pris ensemble représente :

- un composé cyclique saturé à 5 chaînons,
- un composé cyclique saturé à 5 chaînons accolé à un cycle aromatique ;
- un composé cyclique saturé à 6 chaînons ; ou
- un composé hétérocyclique saturé à 6 chaînons comprenant 1 hétéroatome, en position 4, choisi parmi l'oxygène, l'azote et le soufre ;

c) $R_4$ représente un groupement alkyle linéaire ou ramifié de 1 à 6 atomes de carbone substitué par:

- un groupement phényle ; ou
- un groupement phényle substitué par :

- un ou plusieurs halogènes pris parmi le fluor ou le brome ;
- un groupement phényle ou thiényle ;

d) $\mathbf{R_5}$ représente un groupement hydroxyle.

**6.** Composé selon l'une des revendications précédentes dans lequel $\mathbf{R_2}$ représente un groupement isobutyle ou un groupement methyle substitué par:

- un groupement phényle;
- un groupement phényle substitué en position 4 par un halogène pris parmi le fluor ou le brome;
- un groupement phényle substitué en position 4 par un groupement

phényle; et $\mathbf{R_3}$ représente un hydrogène, ou
$\mathbf{R_2}$ et $\mathbf{R_3}$ sont identiques et représentent un groupement méthyle ou éthyle, ou
**-C(R$_2$)(R$_3$)-** représentent ensemble:

- un groupement cyclique saturé à 5 ou 6 chaînons; ou
- un groupement cyclique saturé à 5 chaînons accolé à un cycle aromatique.

**7.** Composé selon l'une des revendications précédentes dans lequel $\mathbf{R_4}$ représente un groupement alkyle à un carbone substitué par:

- un groupement phényle;
- un groupement phényle substitué en position 4 par un halogène pris parmi le fluor ou le brome;
- un groupement phényle substitué en position 4 par un groupement phényle.

**8.** Composé selon l'une des revendications précédentes dans lequel:

- $\mathbf{R_2}$ = $CH_2Ph$; $\mathbf{R_3}$ = H; $\mathbf{R_4}$ = $CH_2Ph$ ; ou
- $\mathbf{R_2}$ = iBu; $\mathbf{R_3}$ = H; $\mathbf{R_4}$ = $CH_2$(4-Br-Ph); ou
- $\mathbf{R_2}$ = $CH_2$(4-Br-Ph); $\mathbf{R_3}$ = H; $\mathbf{R_4}$ = $CH_2$(4-Br-Ph); ou
- $\mathbf{R_2}$ = $CH_2$(4-Br-Ph); $\mathbf{R_3}$ = H; $\mathbf{R_4}$ = $CH_2Ph$;
- $\mathbf{R_2}$ = $CH_2Ph$; $\mathbf{R_3}$ = H; $\mathbf{R_4}$ = $CH_2$(4-Br-Ph); ou
- $\mathbf{R_2}$ = $CH_2$(4-Ph-Ph); $\mathbf{R_3}$ = H; $\mathbf{R_4}$ = $CH_2$(4-Br-Ph); ou
- $\mathbf{R_2}$ = $CH_3$; $\mathbf{R_3}$ = $CH_3$; $\mathbf{R_4}$ = $CH_2$(4-Br-Ph); ou
- $\mathbf{R_2}$ = $C_2H_5$, $\mathbf{R_3}$ = $C_2H_5$; $\mathbf{R_4}$ = $CH_2$(4-Br-Ph); ou
- **-C(R$_2$)(R$_3$)** = cyclopentyle; $\mathbf{R_4}$ = $CH_2$(4-Br-Ph); ou
- **C(R$_2$)(R$_3$)** = cyclohexyle; $\mathbf{R_4}$ = $CH_2$(4-Br-Ph); ou
- **C(R$_2$)(R$_3$)** = indanyle; $\mathbf{R_4}$ = $CH_2$(4-Br-Ph); ou
- **C(R$_2$)(R$_3$)** = cyclohexyle $_0$; $\mathbf{R_4}$ = $CH_2$(4-Ph-Ph); ou
- **C(R$_2$)(R$_3$)** = indanyle; $\mathbf{R_4}$ = $CH_2$(4-Ph-Ph).

**9.** Composé selon l'une des revendications précédentes dans lequel $\mathbf{R_1}$ est choisi parmi-$CH_2CH_2SCH_3$, $CH_2CH_2SOCH_3$,-$CH_2CH_2CH_2CH_3$.

**10.** Composé selon l'une des revendications 1 à 7 dans lequel:

- $\mathbf{R_1}$ = $CH_2CH_2SCH_3$; $\mathbf{R_2}$ = $CH_2CH(CH_3)_2$; $\mathbf{R_3}$= H; $\mathbf{R_4}$ = $CH_2$(4-Br-Ph);
- $\mathbf{R_1}$ = $CH_2CH_2CH_2CH_3$; $\mathbf{R_2}$ = $CH_2CH(CH_3)_2$; $\mathbf{R_3}$= H; $\mathbf{R_4}$ = $CH_2$(4-Br-Ph);
- $\mathbf{R_1}$ = $CH_2CH_2SOCH_3$; $\mathbf{R_2}$ = $CH_2CH(CH_3)_2$; $\mathbf{R_3}$= H; $\mathbf{R_4}$ = $CH_2$(4-Br-Ph);
- $\mathbf{R_1}$ = $CH_2CH_2CH_2CH_3$; **C(R$_2$)(R$_3$)-** = Cyclohexyle; $\mathbf{R_4}$ = $CH_2$(4-Br-Ph);
- $\mathbf{R_1}$ = $CH_2CH_2SCH_3$; **C(R$_2$)(R$_3$)** = Cyclohexyle; $\mathbf{R_4}$ = $CH_2$(4-Ph-Ph);
- $\mathbf{R_1}$ = $CH_2CH_2CH_2CH_3$; **C(R$_2$)(R$_3$)** = Cyclohexyle; $\mathbf{R_4}$ = $CH_2$(4-Ph-Ph).

**11.** Composé selon l'une des revendications précédentes pour utilisation en tant que médicaments.

**12.** Composé selon la revendication 11 pour son utilisation en tant que analgésique, anxiolytique, antidépresseur ou antiinflammatoire.

**13.** Composition pharmaceutique comprenant au moins un composé selon l'une des revendications 1 à 10 et au moins un excipient pharmaceutiquement acceptable.

**14.** Composition pharmaceutique selon la revendication 13 pour son utilisation en tant que analgésique, anxiolytique, antidépresseur ou antiinflammatoire.

**15.** Composition pharmaceutique selon la revendication 13 ou 14 comprenant au moins un composé choisi parmi la morphine et ses dérivés, les endocannabinoïdes et les inhibiteurs de leur métabolisme, les dérivés du GABA tels que la gabapentine ou la prégabaline, la duloxetine ou la méthadone.

**16.** Composition pharmaceutique selon l'une des revendications 13 à 15 destinée à être administrée par voie parentérale, topique, orale, ou nasale.

**Patentansprüche**

**1.** Verbindung mit der allgemeinen Formel (1)

(1) $R\text{-}NH\text{-}CH(R_1)\text{-}CH_2\text{-}S\text{-}S\text{-}C(R_2)(R_3)\text{-}COCH_2\text{-}CH(R_4)\text{-}COR_5$,

wobei

a) R Folgendes darstellt:

- einen Wasserstoff;
- eine R'C(O)OCH(R")OC(O)-Alkoxyalkylcarbonyl-Gruppe, wobei R' und R" unabhängig voneinander eine Alkylgruppe darstellen, die 1 bis 6 Kohlenstoffatome enthält;

b) $R_1$ eine lineare oder verzweigte Alkylgruppe mit 1 bis 6 Kohlenstoffatomen darstellt, die durch eine-OR''', -SOR''' oder -SR''' Gruppe substituiert ist oder nicht, wobei R''' eine Alkylgruppe mit 1 bis 6 Kohlenstoffatomen darstellt, die durch ein oder mehrere Halogenatome substituiert ist oder nicht;
c) $R_2$ Folgendes darstellt:

- eine lineare oder verzweigte Alkylgruppe mit 1 bis 6 Kohlenstoffatomen, die durch Folgendes substituiert ist oder nicht:

  - eine $-OR_6$, $-SR_6$ oder $-SOR_6$ Gruppe, wobei $R_6$ einen Wasserstoff, eine lineare oder verzweigte Alkylgruppe mit 1 bis 4 Kohlenstoffen, eine Phenyl- oder Benzylgruppe darstellt;
  - eine $-CO_2R_7$ Gruppe, wobei $R_7$ einen Wasserstoff, eine lineare oder verzweigte Alkylgruppe, die 2 bis 4 Kohlenstoffatome umfasst, oder eine Benzylgruppe darstellt;
  - eine $-NR_8R_9$ Gruppe, wobei $R_8$ und $R_9$ unabhängig voneinander einen Wasserstoff, eine lineare oder verzweigte Alkylgruppe mit 1 bis 4 Kohlenstoffatomen, eine Phenyl- oder Benzylgruppe darstellen, oder mit $-NR_8R_9$, die zusammen genommen eine gesättigte heterocyclische 5- oder 6-gliedrige Verbindung darstellen, die ein oder mehrere Heteroatome umfasst, die unter N oder O ausgewählt sind, wobei sie vorzugsweise ein Morpholin oder ein Piperidin darstellt; oder
  - eine $-CONR_8R_9$ Carboxamidgruppe mit $-NR_8R_9$, wie vorhergehend definiert;
  - eine Phenylgruppe, die durch ein oder mehrere Halogene, die unter Fluor oder Brom genommen sind, eine $-OR_6$-Alkoxygruppe, wobei $R_6$ die gleiche Definition wie vorhergehend aufweist, oder durch eine Phenylgruppe substituiert ist oder nicht;
  - eine aromatische heterocyclische 5- oder 6-gliedrige Verbindung, die 1 oder 2 Heteroatome umfasst, das/die unter Sauerstoff, Stickstoff und Schwefel ausgewählt ist/sind;
  - eine gesättigte cyclische 5- oder 6-gliedrige oder gesättigte heterozyklische 5- oder 6-gliedrige Verbindung, die 1 oder 2 Heteroatome umfasst, die unter Sauerstoff, Stickstoff und Schwefel ausgewählt sind;

- eine gesättigte cyclische 5- oder 6-gliedrige oder gesättigte heterozyklische 5- oder 6-gliedrige Verbindung, die 1 oder 2 Heteroatome umfasst, die unter Sauerstoff, Stickstoff und Schwefel ausgewählt sind; oder
- eine Phenylgruppe, die durch ein oder mehrere Halogene, die unter Fluor oder Brom ausgewählt sind,

oder durch eine -$OR_5$ Gruppe substituiert ist oder nicht, wobei $R_5$ die gleiche Definition wie vorhergehend aufweist;
und $R_3$ einen Wasserstoff darstellt; oder
$R_2$ und $R_3$ identisch sind und eine lineare oder verzweigte Alkylgruppe mit 1 bis 6 Kohlenstoffatomen darstellen; oder
-$C(R_2)$ $(R_3)$- zusammen genommen Folgendes darstellt:

- eine gesättigte cyclische Verbindung mit 5 Gliedern, die mit einen aromatischen Ring kondensiert ist oder nicht, was vorzugsweise zum Indanylring führt;
- eine gesättigte cyclische Verbindung mit 6 Gliedern;
- eine gesättigte heterocyclische Verbindung mit 6 Gliedern, die 1 Heteroatom an der Position 4 umfasst, das unter Sauerstoff, Stickstoff und Schwefel ausgewählt ist, wobei, wenn das Heteroatom Stickstoff ist, der Stickstoff durch eine Alkylgruppe mit 1 bis 6 Kohlenstoffatomen, eine Phenyl-, Benzyl- oder eine Alkanoylgruppe substituiert ist oder nicht;

d) $R_4$ Folgendes darstellt:

- eine lineare oder verzweigte Alkylgruppe mit 1 bis 6 Kohlenstoffatomen, die durch Folgendes substituiert ist oder nicht;

  - eine -$OR_6$, -$SR_6$ oder -$SOR_6$ Gruppe, wobei $R_6$ einen Wasserstoff, eine lineare oder verzweigte Alkylgruppe mit 1 bis 4 Kohlenstoffen, eine Phenyl- oder Benzylgruppe darstellt;
  - eine -$CO_2R_7$ Gruppe, wobei $R_7$ einen Wasserstoff, eine lineare oder verzweigte Alkylgruppe mit 2 bis 4 Kohlenstoffatomen, eine Benzylgruppe darstellt;
  - eine -$NR_8R_9$ Gruppe, wobei $R_8$ und $R_9$ unabhängig voneinander einen Wasserstoff, eine lineare oder verzweigte Alkylgruppe mit 1 bis 4 Kohlenstoffatomen, eine Phenyl- oder Benzylgruppe oder mit -$NR_8R_9$ darstellen, die zusammen genommen eine gesättigte heterocyclische 5- oder 6-gliedrige Verbindung darstellen, die ein oder mehrere Heteroatome umfasst, die unter N oder O ausgewählt sind, wobei sie vorzugsweise ein Morpholin oder ein Piperidin darstellt;
  - eine -$CONR_8R_9$ Carboxamid-Gruppe mit -$NR_8R_9$, wie vorhergehend definiert;
  - eine Phenylgruppe, die durch Folgendes substituiert ist oder nicht:

    - ein oder mehrere Halogene, die unter Fluor oder Brom ausgewählt sind;
    - eine -$OR_6$ Gruppe, wobei $R_6$ die gleiche Definition wie vorhergehend aufweist;
    - eine Phenyl- oder Thienylgruppe;

  - eine aromatische heterocyclische 5- oder 6-gliedrige Verbindung, die 1 oder 2 Heteroatome umfasst, das/die unter Sauerstoff, Stickstoff und Schwefel ausgewählt ist/sind; oder
  - eine gesättigte cyclische 5- oder 6-gliedrige oder gesättigte heterozyklische 5- oder 6-gliedrige Verbindung, die 1 oder 2 Heteroatome umfasst, die unter Sauerstoff, Stickstoff und Schwefel ausgewählt sind;

- eine Phenylgruppe, die durch Folgendes substituiert ist oder nicht:

  - ein oder mehrere Halogene, insbesondere Fluor oder Brom;
  - eine -$OR_6$ Gruppe, wobei $R_6$ die gleiche Definition wie vorhergehend aufweist;
  - ein Phenyl;
  - eine aromatische heterocyclische 5- oder 6-gliedrige Verbindung, die 1 oder 2 Heteroatome umfasst, die unter Sauerstoff, Stickstoff und Schwefel ausgewählt sind;

c) $R_5$ Folgendes darstellt:

- eine Hydroxylgruppe;
- eine -$NR_8R_9$ Gruppe, wobei $R_8$ und $R_9$ unabhängig voneinander einen Wasserstoff, eine lineare oder verzweigte Alkylgruppe mit 1 bis 4 Kohlenstoffatomen, eine Phenyl- oder Benzylgruppe oder mit -$NR_8R_9$ darstellen, die zusammen genommen eine heterocyclische 5- oder 6-gliedrige Verbindung darstellen, die ein oder mehrere Heteroatome umfasst, die unter N oder O ausgewählt sind, wobei sie vorzugsweise ein Morpholin oder ein Piperidin darstellt;

- eine $-OR_{10}$ Alkoxygruppe, wobei $R_{10}$ Folgendes darstellt:

  - eine lineare oder verzweigte Alkylgruppe, die 2 bis 6 Kohlenstoffatome umfasst;
  - eine Benzylgruppe;
  - eine $-CHR_{11}-COOR_{12}$, $-CHR_{11}-O-C(=O)R_{12}$, $-CHR_{11}$ oder $-C(=O)-OR_{12}$ Gruppe, wobei $R_{11}$ und $R_{12}$ unabhängig voneinander eine lineare oder verzweigte Alkylgruppe mit 1 bis 6 Kohlenstoffatomen darstellen.

**2.** Verbindung nach Anspruch 1, wobei $R_2$ eine lineare oder verzweigte Alkylgruppe mit 1 bis 6 Kohlenstoffatomen oder eine lineare oder verzweigte Alkylgruppe mit 1 bis 6 Kohlenstoffatomen darstellt, die durch Folgendes substituiert ist:

- eine Phenylgruppe;
- eine Phenylgruppe, die durch ein oder mehrere Halogene substituiert ist, die unter Fluor oder Brom genommen sind;
- eine aromatische heterocyclische 5- oder 6-gliedrige Verbindung, die 1 oder 2 Heteroatome umfasst, das/die unter Sauerstoff, Stickstoff und Schwefel ausgewählt ist/sind;
- eine gesättigte cyclische 5- oder 6-gliedrige oder gesättigte heterozyklische 5- oder 6-gliedrige Verbindung, die 1 oder 2 Heteroatome umfasst, die unter Sauerstoff, Stickstoff und Schwefel ausgewählt sind;

und $R_3$ einen Wasserstoff darstellt, oder
$R_2$ und $R_3$ identisch sind und eine lineare oder verzweigte Alkylgruppe mit 1 bis 6 Kohlenstoffatomen darstellen; oder
$-C(R_2)(R_3)-$ zusammen genommen Folgendes darstellt:

- eine gesättigte cyclische Verbindung mit 5 Gliedern,
- eine gesättigte cyclische Verbindung mit 5 Gliedern, die mit einem aromatischen Ring kondensiert ist;
- eine gesättigte cyclische Verbindung mit 6 Gliedern; oder
- eine gesättigte heterocyclische Verbindung mit 6 Gliedern, die 1 Heteroatom an der Position 4 umfasst, das unter Sauerstoff, Stickstoff und Schwefel ausgewählt ist.

**3.** Verbindung nach Anspruch 1 oder 2, wobei $R_4$ eine lineare oder verzweigte Alkylgruppe mit 1 bis 6 Kohlenstoffatomen darstellt, die durch Folgendes substituiert ist:

- eine Phenylgruppe; oder
- eine Phenylgruppe, die durch Folgendes substituiert ist:

  - ein oder mehrere Halogene, die unter Fluor oder Brom genommen sind;
  - eine Phenyl- oder Thienylgruppe.

**4.** Verbindung nach einem der vorhergehenden Ansprüche, wobei $R_5$ ein Hydroxyl darstellt.

**5.** Verbindung nach einem der vorhergehenden Ansprüche, wobei:

a) $R_1$ unter $-CH_2CH_2SCH_3$, $-CH_2CH_2SOCH_3$, $-CH_2CH_2CH_2CH_3$ ausgewählt ist;
b) $R_2$ eine lineare oder verzweigte Alkylgruppe mit 1 bis 6 Kohlenstoffatomen oder eine lineare oder verzweigte Alkylgruppe mit 1 bis 6 Kohlenstoffatomen darstellt, die durch Folgendes substituiert ist:

- eine Phenylgruppe;
- eine Phenylgruppe, die durch ein oder mehrere Halogene substituiert ist, die unter Fluor oder Brom genommen sind;
- eine aromatische heterocyclische 5- oder 6-gliedrige Verbindung, die 1 oder 2 Heteroatome umfasst, das/die unter Sauerstoff, Stickstoff und Schwefel ausgewählt ist/sind;
- eine gesättigte cyclische 5- oder 6-gliedrige oder gesättigte heterozyklische 5- oder 6-gliedrige Verbindung, die 1 oder 2 Heteroatome umfasst, die unter Sauerstoff, Stickstoff und Schwefel ausgewählt sind;

und $R_3$ einen Wasserstoff darstellt, oder
$R_2$ und $R_3$ identisch sind und eine lineare oder verzweigte Alkylgruppe mit 1 bis 6 Kohlenstoffatomen darstellen; oder
$-C(R_2)(R_3)-$ zusammen genommen Folgendes darstellt:

- eine gesättigte cyclische Verbindung mit 5 Gliedern,
- eine gesättigte cyclische Verbindung mit 5 Gliedern, die mit einem aromatischen Ring kondensiert ist;
- eine gesättigte cyclische Verbindung mit 6 Gliedern; oder
- eine gesättigte heterocyclische Verbindung mit 6 Gliedern, die 1 Heteroatom an der Position 4 umfasst, das unter Sauerstoff, Stickstoff und Schwefel ausgewählt ist;

c) $R_4$ eine lineare oder verzweigte Alkylgruppe mit 1 bis 6 Kohlenstoffatomen darstellt, die durch Folgendes substituiert ist:

- eine Phenylgruppe; oder
- eine Phenylgruppe, die durch Folgendes substituiert ist:
  - ein oder mehrere Halogene, die unter Fluor oder Brom genommen sind;
  - eine Phenyl- oder Thienylgruppe;

d) $R_5$ eine Hydroxylgruppe darstellt.

**6.** Verbindung nach einem der vorhergehenden Ansprüche, wobei $R_2$ eine Isobutylgruppe oder eine Methylgruppe darstellt, die durch Folgendes substituiert ist:

- eine Phenylgruppe;
- eine Phenylgruppe, die an der Position 4 durch ein Halogen substituiert ist, das unter Fluor oder Brom ausgewählt ist;
- eine Phenylgruppe, die an der Position 4 durch eine Phenylgruppe substituiert ist;

und $R_3$ einen Wasserstoff darstellt, oder
$R_2$ und $R_3$ identisch sind und eine Methyl- oder Ethylgruppe darstellen, oder
$-C(R_2)$ $(R_3)$- zusammen Folgendes darstellen:

- eine gesättigte cyclische 5- oder 6-gliedrige Gruppe; oder
- eine gesättigte cyclische Gruppe mit 5 Gliedern, die mit einem aromatischen Ring kondensiert ist.

**7.** Verbindung nach einem der vorhergehenden Ansprüche, wobei $R_4$ eine Alkylgruppe mit einem Kohlenstoff darstellt, die durch Folgendes substituiert ist:

- eine Phenylgruppe;
- eine Phenylgruppe, die an der Position 4 durch ein Halogen substituiert ist, das unter Fluor oder Brom genommen ist;
- eine Phenylgruppe, die an der Position 4 durch eine Phenylgruppe substituiert ist.

**8.** Verbindung nach einem der vorhergehenden Ansprüche, wobei:

- $R_2 = CH_2Ph$; $R_3 = H$; $R_4 = CH_2Ph$; oder
- $R_2 = iBu$; $R_3 = H$; $R_4 = CH_2$ (4-Br-Ph) ; oder
- $R_2 = CH_2$ (4-Br-Ph) ; $R_3 = H$; $R_4 = CH_2$ (4-Br-Ph) ; oder
- $R_2 = CH_2$ (4-Br-Ph) ; $R_3 = H$; $R_4 = CH_2Ph$;
- $R_2 = CH_2Ph$; $R_3 = H$; $R_4 = CH_2$ (4-Br-Ph) ; oder
- $R_2 = CH_2$ (4-Ph-Ph) ; $R_3 = H$; $R_4 = CH_2$ (4-Br-Ph) ; oder
- $R_2 = CH_3$; $R_3 = CH_3$; $R_4 = CH_2$ (4-Br-Ph) ; oder
- $R_2 = C_2H_5$; $R_3 = C_2H_5$; $R_4 = CH_2$(4-Br-Ph) ; oder
- -$C(R_2)(R_3)$ = Cyclopentyl; $R_4 = CH_2$(4-Br-Ph) ; oder
- $C(R_2)(R_3)$ = Cyclohexyl; $R_4 = CH_2$(4-Br-Ph) ; oder
- $C(R_2)(R_3)$ = Indanyl; $R_4 = CH_2$(4-Br-Ph) ; oder
- $C(R_2)(R_3)$ = Cyclohexyl; $R_4 = CH_2$(4-Ph-Ph) ; oder
- $C(R_2)(R_3)$ = Indanyl $R_4 = CH_2$(4-Ph-Ph)

**9.** Verbindung nach einem der vorhergehenden Ansprüche, wobei $R_1$ unter Folgendem ausgewählt ist: $-CH_2CH_2SCH_3$, $CH_2CH_2SOCH_3$, $-CH_2CH_2CH_2CH_3$.

**10.** Verbindung nach einem der Ansprüche 1 bis 7, wobei:

- $R_1 = CH_2CH_2SCH_3$; $R_2 = CH_2CH(CH_3)_2$; $R_3$= H; $R_4 = CH_2$(4-Br-Ph) ;
- $R_1 = CH_2CH_2CH_2CH_3$; $R_2 = CH_2CH(CH_3)_2$; $R_3$= H; R4 = $CH_2$(4-Br-Ph) ;
- $R_1 = CH_2CH_2SOCH_3$; $R_2 = CH_2CH(CH_3)_2$; $R_3$= H; $R_4 = CH_2$(4-Br-Ph) ;
- $R_1 = CH_2CH_2CH_2CH_3$; $C(R_2)(R_3)$- = Cyclohexyl; $R_4 = CH_2$(4-Br-Ph) ;
- $R_1 = CH_2CH_2SCH_3$; $C(R_2)(R_3)$ = Cyclohexyl; $R_4 = CH_2$(4-Ph-Ph);
- $R_1 = CH_2CH_2CH_2CH_3$; $C(R_2)(R_3)$ = Cyclohexyl; $R_4 = CH_2$(4-Ph-Ph).

**11.** Verbindung nach einem der vorhergehenden Ansprüche zur Verwendung als Medikament.

**12.** Verbindung nach Anspruch 11 zur Verwendung als Analgetikum, Anxiolytikum, Antidepressivum oder Entzündungshemmer.

**13.** Pharmazeutische Zusammensetzung, die mindestens eine Verbindung nach einem der Ansprüche 1 bis 10 und mindestens einen pharmazeutisch annehmbaren Träger umfasst.

**14.** Pharmazeutische Zusammensetzung nach Anspruch 13 zur Verwendung als Analgetikum, Anxiolytikum, Antidepressivum oder Entzündungshemmer.

**15.** Pharmazeutische Zusammensetzung nach Anspruch 13 oder 14, die mindestens eine Verbindung umfasst, die unter Morphin und seinen Derivaten, Endocannabinoiden und Hemmern ihres Metabolismus, GABA-Derivaten, wie Gabapentin oder Pregabalin, Duloxetin oder Methadon ausgewählt ist.

**16.** Pharmazeutische Zusammensetzung nach einem der Ansprüche 13 bis 15, die dazu bestimmt ist, auf parenteralem, topischem, oralem oder nasalem Weg verabreicht zu werden.

## Claims

**1.** A compound of the general formula (1)

(1) $R\text{-}NH\text{-}CH(R_1)\text{-}CH_2\text{-}S\text{-}S\text{-}C(R_2)(R_3)\text{-}COCH_2\text{-}CH(R_4)\text{-}COR_5$

wherein

a) R is:

- hydrogen;
- an alkoxyalkylcarbonyl group R'C(O)OCH(R")OC(O)- where R' and R" are, independently, an alkyl group containing 1 to 6 carbon atoms;

b) $R_1$ is a linear or branched alkyl group of 1 to 6 carbon atoms, substituted, or not, by an -OR''', -SOR''' or -SR''' group, with R''' being an alkyl group of 1 to 6 carbon atoms, substituted, or not, by one or more halogen atoms;

c) $R_2$ is:

- a linear or branched alkyl group of 1 to 6 carbon atoms, substituted or not, by:
  - an $-OR_6$, $-SR_6$ or $-SOR_6$ group, with $R_6$ being hydrogen, a linear or branched alkyl group of 1 to 4 carbons, a phenyl or benzyl group;
  - a $-CO_2R_7$ group, with $R_7$ being hydrogen, a linear or branched alkyl group comprising 2 to 4 carbon atoms, or a benzyl group;
  - an $-NR_8R_9$ group, with $R_8$ and $R_9$ being, independently, hydrogen, a linear or branched alkyl group of 1 to 4 carbon atoms, a phenyl or benzyl group, or with $-NR_8R_9$ taken together being a saturated 5- or 6-membered heterocycle comprising one or more heteroatoms selected from N or O, preferably being a morpholine or a piperidine; or
  - a carboxamide group $-CONR_8R_9$, with $-NR_8R_9$ as defined above;

- a phenyl group, substituted, or not, by one or more halogens selected from fluorine or bromine, an alkoxy group $-OR_6$, with $R_6$ having the same definition as above, or by a phenyl group;
- an aromatic 5- or 6-membered heterocycle comprising 1 or 2 heteroatoms selected from oxygen, nitrogen and sulfur;
- a saturated 5- or 6-membered cyclic or heterocyclic compound comprising 1 or 2 heteroatoms selected from oxygen, nitrogen and sulfur;

- a saturated 5- or 6-membered cyclic or heterocyclic compound comprising 1 or 2 heteroatoms selected from oxygen, nitrogen and sulfur; or
- a phenyl group, substituted or not, by one or more halogens selected from fluorine or bromine, or by an $-OR_5$ group with $R_5$ having the same definition as above;
and $R_3$ is hydrogen; or
$R_2$ and $R_3$ are identical and are a linear or branched alkyl group of 1 to 6 carbon atoms; or
$-C(R_2)$ $(R_3)$- taken together is:

- a saturated 5-membered cyclic compound fused, or not, to an aromatic ring, preferably leading to the indanyl ring;
- a saturated 6-membered cyclic compound;
- a saturated 6-membered heterocyclic compound comprising 1 heteroatom, at the 4-position, selected from oxygen, nitrogen and sulfur, wherein when the heteroatom is nitrogen, the nitrogen is substituted, or not, by an alkyl group of 1 to 6 carbon atoms, a phenyl, benzyl or alkanoyl group;

d) $R_4$ is:

- a linear or branched alkyl group of 1 to 6 carbon atoms, substituted, or not, by:

  - an $-OR_6$, $-SR_6$ or $-SOR_6$ group, with $R_6$ being hydrogen, a linear or branched alkyl group of 1 to 4 carbons, a phenyl or benzyl group;
  - a $-CO_2R_7$ group, with $R_7$ being hydrogen, a linear or branched alkyl group comprising 2 to 4 carbon atoms, a benzyl group;
  - an $-NR_8R_9$ group, with $R_8$ and $R_9$ being, independently, hydrogen, a linear or branched alkyl group of 1 to 4 carbon atoms, a phenyl or benzyl group, or with $-NR_8R_9$ taken together being a saturated 5- or 6-membered heterocycle comprising one or more heteroatoms selected from N or O, preferably being a morpholine or a piperidine;
  - a carboxamide group $-CONR_8R_9$, with $-NR_8R_9$ as defined above;
  - a phenyl group substituted, or not, by:

    - one or more halogens selected from fluorine or bromine;
    - an $-OR_6$ group, $R_6$ having the same definition as above;
    - a phenyl or thienyl group;

  - an aromatic 5- or 6-membered heterocycle comprising 1 or 2 heteroatoms selected from oxygen, nitrogen and sulfur; or
  - a saturated 5- or 6-membered cyclic or heterocyclic compound comprising 1 or 2 heteroatoms selected from oxygen, nitrogen and sulfur;

- a phenyl group, substituted or not, by:

  - one or more halogens, notably fluorine or bromine;
  - an $-OR_6$ group, with $R_6$ having the same definition as above;
  - a phenyl;
  - an aromatic 5- or 6-membered heterocycle comprising 1 or 2 heteroatoms selected from oxygen, nitrogen and sulfur;

e) $R_5$ is:

- a hydroxyl group;
- an $-NR_8R_9$ group, with $R_8$ and $R_9$ being, independently, hydrogen, a linear or branched alkyl group of 1

to 4 carbon atoms, a phenyl or benzyl group, or with $-NR_8R_9$ taken together being a 5- or 6-membered heterocycle, comprising one or more heteroatoms selected from N or O, preferably being a morpholine or a piperidine;
- an alkoxy group $-OR_{10}$, with $R_{10}$ being:

  - a linear or branched alkyl group, comprised of 2 to 6 carbon atoms;
  - a benzyl group;
  - a $-CHR_{11}-COOR_{12}$, $-CHR_{11}-O-C(=O)R_{12}$, $-CHR_{11}-$ or $-C(=O)-OR_{12}$ group wherein $R_{11}$ and $R_{12}$ are, independently, a linear or branched alkyl group of 1 to 6 carbon atoms.

**2.** The compound according to claim 1, wherein $R_2$ is a linear or branched alkyl group of 1 to 6 carbon atoms or a linear or branched alkyl group of 1 to 6 carbon atoms substituted by:

- a phenyl group;
- a phenyl group substituted by one or more halogens selected from fluorine or bromine;
- an aromatic 5- or 6-membered heterocycle comprising 1 or 2 heteroatoms selected from oxygen, nitrogen and sulfur;
- a saturated 5- or 6-membered cyclic or heterocyclic compound comprising 1 or 2 heteroatoms selected from oxygen, nitrogen and sulfur;

and $R_3$ is hydrogen, or
$R_2$ and $R_3$ are identical and are a linear or branched alkyl group of 1 to 6 carbon atoms, or
$-C(R_2)(R_3)-$ taken together is:

- a saturated 5-membered cyclic compound;
- a saturated 5-membered cyclic compound fused to an aromatic ring;
- a saturated 6-membered cyclic compound; or
- a saturated 6-membered heterocyclic compound comprising 1 heteroatom, at the 4-position, selected from oxygen, nitrogen and sulfur.

**3.** The compound according to claim 1 or 2, wherein $R_4$ is a linear or branched alkyl group of 1 to 6 carbon atoms substituted by:

- a phenyl group; or
- a phenyl group substituted by:

  - one or more halogens selected from fluorine or bromine;
  - a phenyl or thienyl group.

**4.** The compound according to any one of the preceding claims, wherein $R_5$ is a hydroxyl.

**5.** The compound according to any one of the preceding claims, wherein:

a) $R_1$ is selected from $-CH_2CH_2SCH_3$, $-CH_2CH_2SOCH_3$, $-CH_2CH_2CH_2CH_3$;
b) $R_2$ is a linear or branched alkyl group of 1 to 6 carbon atoms or a linear or branched alkyl group of 1 to 6 carbon atoms substituted by:

  - a phenyl group;
  - a phenyl group substituted by one or more halogens selected from fluorine or bromine;
  - an aromatic 5- or 6-membered heterocycle comprising 1 or 2 heteroatoms selected from oxygen, nitrogen and sulfur;
  - a saturated 5- or 6-membered cyclic or heterocyclic compound comprising 1 or 2 heteroatoms selected from oxygen, nitrogen and sulfur;

and $R_3$ is hydrogen, or
$R_2$ and $R_3$ are identical and are a linear or branched alkyl group of 1 to 6 carbon atoms; or
$-C(R_2)(R_3)-$ taken together is:

- a saturated 5-membered cyclic compound;
- a saturated 5-membered cyclic compound fused to an aromatic ring;
- a saturated 6-membered cyclic compound; or
- a saturated 6-membered heterocyclic compound comprising 1 heteroatom, at the 4-position, selected from oxygen, nitrogen and sulfur;

c) $R_4$ is a linear or branched alkyl group of 1 to 6 carbon atoms substituted by:

- a phenyl group; or
- a phenyl group substituted by:
    - one or more halogens selected from fluorine or bromine;
    - a phenyl or thienyl group;

d) $R_5$ is a hydroxyl group.

**6.** The compound according to any one of the preceding claims, wherein $R_2$ is an isobutyl group or a methyl group substituted by:

- a phenyl group;
- a phenyl group substituted at the 4-position by a halogen selected from fluorine or bromine;
- a phenyl group substituted at the 4-position by a phenyl group;

and $R_3$ is hydrogen, or
$R_2$ and $R_3$ are identical and are a methyl or ethyl group, or
-C($R_2$) ($R_3$)- are together:

- a saturated 5- or 6-membered cyclic group; or
- a saturated 5-membered cyclic group fused to an aromatic ring.

**7.** The compound according to any one of the preceding claims, wherein $R_4$ is an alkyl group with one carbon substituted by:

- a phenyl group;
- a phenyl group substituted at the 4-position by a halogen selected from fluorine or bromine;
- a phenyl group substituted at the 4-position by a phenyl group.

**8.** The compound according to any one of the preceding claims, wherein:

- $R_2$ = $CH_2$Ph; $R_3$ = H; $R_4$ = $CH_2$Ph; or
- $R_2$ = iBu; $R_3$ = H; $R_4$ = $CH_2$(4-Br-Ph) ; or
- $R_2$ = $CH_2$(4-Br-Ph) ; $R_3$ = H; $R_4$ = $CH_2$(4-Br-Ph) ; or
- $R_2$ = $CH_2$(4-Br-Ph) ; $R_3$ = H; $R_4$ = $CH_2$Ph;
- $R_2$ = $CH_2$Ph; $R_3$ = H; $R_4$ = $CH_2$(4-Br-Ph) ; or
- $R_2$ = $CH_2$(4-Ph-Ph) ; $R_3$ = H; $R_4$ = $CH_2$(4-Br-Ph) ; or
- $R_2$ = $CH_3$; $R_3$ = $CH_3$; $R_4$ = $CH_2$(4-Br-Ph) ; or
- $R_2$ = $C_2H_5$; $R_3$ = $C_2H_5$; $R_4$ = $CH_2$(4-Br-Ph) ; or
- -C($R_2$)($R_3$) = cyclopentyl; $R_4$ = $CH_2$(4-Br-Ph) ; or
- C($R_2$)($R_3$) = cyclohexyl; $R_4$ = $CH_2$(4-Br-Ph) ; or
- C($R_2$)($R_3$) = indanyl; $R_4$ = $CH_2$(4-Br-Ph) ; or
- C($R_2$)($R_3$) = cyclohexyl; $R_4$ = $CH_2$(4-Ph-Ph) ; or
- C($R_2$)($R_3$) = indanyl; $R_4$ = $CH_2$(4-Br-Ph).

**9.** The compound according to any one of the preceding claims, wherein $R_1$ is selected from $-CH_2CH_2SCH_3$, $CH_2CH_2SOCH_3$, $-CH_2CH_2CH_2CH_3$.

**10.** The compound according to any one of claims 1 to 7 wherein:

- $R_1 = CH_2CH_2SCH_3$; $R_2 = CH_2CH(CH_3)_2$; $R_3$ = H; $R_4 = CH_2$(4-Br-Ph) ;
- $R_1 = CH_2CH_2CH_2CH_3$; $R_2 = CH_2CH(CH_3)_2$; $R_3$ = H; $R_4 = CH_2$(4-Br-Ph) ;
- $R_1 = CH_2CH_2SOCH_3$; $R_2 = CH_2CH(CH_3)_2$; $R_3$ = H; $R_4 = CH_2$(4-Br-Ph) ;
- $R_1 = CH_2CH_2CH_2CH_3$; $C(R_2)(R_3)$ - = Cyclohexyl; $R_4 = CH_2$(4-Br-Ph) ;
- $R_1 = CH_2CH_2SCH_3$; $C(R_2)(R_3)$ = Cyclohexyl; $R_4 = CH_2$(4-Ph-Ph);
- $R_1 = CH_2CH_2CH_2CH_3$; $C(R_2)(R_3)$ = Cyclohexyl; $R_4 = CH_2$(4-Ph-Ph).

**11.** The compound according to any one of the preceding claims, for use as a drug.

**12.** The compound according to claim 11, for use as an analgesic, anxiolytic, antidepressant or anti-inflammatory.

**13.** A pharmaceutical composition comprising at least one compound according to any one of claims 1 to 10 and at least one pharmaceutically acceptable excipient.

**14.** The pharmaceutical composition according to claim 13, for use as an analgesic, anxiolytic, antidepressant or anti-inflammatory.

**15.** The pharmaceutical composition according to claim 13 or 14, comprising at least one compound selected from morphine and derivatives thereof, endocannabinoids and inhibitors of endocannabinoid metabolism, GABA derivatives such as gabapentin or pregabalin, duloxetine or methadone.

**16.** The pharmaceutical composition according to any one of claims 13 to 15, for parenteral, topical, oral, or nasal administration.

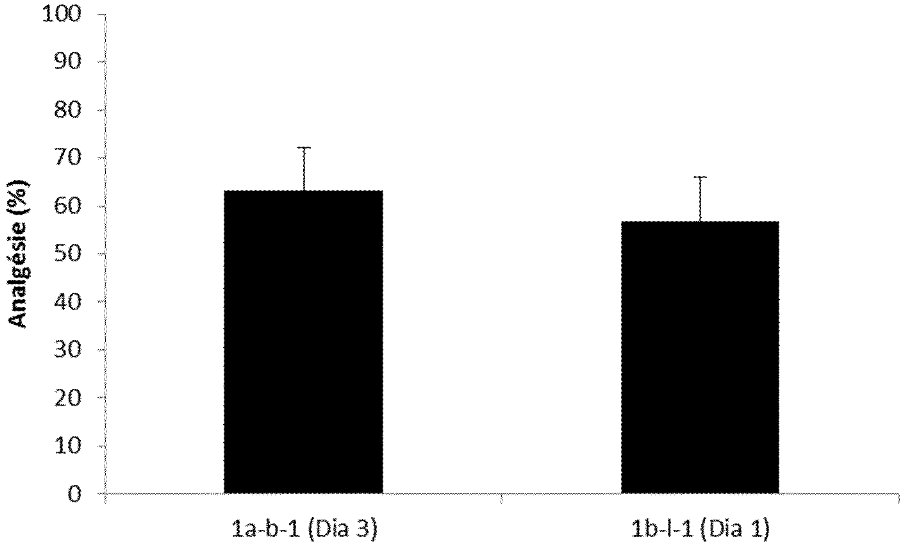


Fig. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2518088
- FR 2605004
- FR 2755135
- FR 2777780
- FR 0855015
- FR 2651229
- FR 0510862
- FR 0604030
- FR 0853092
- WO 2009138436 A
- FR 2892413


### Littérature non-brevet citée dans la description


- **MOSNAIM et al.** *Neurochem. Res.,* 2008, vol. 33, 81-86
- **ROQUES et al.** *Pharmacol. Rev.,* 1993, vol. 45, 87-146
- **NOBLE et al.** *Expert. Opin. Ther. Targets,* 2007, vol. 11, 145-149
- **WISNER et al.** *PNAS,* 2006, vol. 103, 17979-17984
- **MENENDEZ et al.** *Eur J Pharmacol,* 2008, vol. 596, 50-55
- **THIBAULT et al.** *Eur. J. Pharmacol.,* 2008, vol. 600, 71-77
- **FOURNIÉ-ZALUSKI et al.** *J. Med. Chem.,* 1992, vol. 35, 2473-2481
- **ROQUES et al.** *Nature Rev. Drug Discov.,* 2012, vol. 11, 292-311
- **NOBLE et al.** *Journal of Pharmacology and Experimental Therapeutics,* 1992, vol. 261 (1), 181-190
- **NOBLE et al.** *Pain,* 1997, vol. 73 (97), 383-391
- **MAS NIETO et al.** *Neuropharmacol,* 2001, vol. 41, 496-506
- **VALVERDE et al.** *Eur. J. Neurosci.,* 2001, vol. 13, 1816-1824
- **MENENDEZ et al.** *Eur. J. Pharmacol.,* 2007, vol. 596, 50-55
- **LE GUEN et al.** *Pain,* 2003, vol. 104, 139-148
- **FOURNIÉ-ZALUSKI M-C. et al.** *J. Med. Chem.,* 1992, vol. 35, 2473-2481
- **CUNDY et al.** *J. Pharm. Exp. Therap.,* 2004, vol. 311, 315-323
- **CLAESON G. et al.** *Acta Chem. Scand.,* 1968, vol. 22, 3155-3159
- **DUTTA A. et al.** *J. Chem. Soc. Perkin Trans.,* 1987, vol. 1, 111-120
- **CHEN et al.** *Tet. Lett.,* 1997, vol. 38 (18), 3175-3178
- **HARRIS K.M. et al.** *J. Pept. Sci.,* 2007, vol. 2, 81-93
- *J.Med.Chem.,* 1992, vol. 35, 2473
- **BARCELO et al.** *Synthesis,* 1986, 627
- **AUBRY M. et al.** *Biochem. Cell. Biol.,* 1987, vol. 65, 398-404
- **GOUDREAU N. et al.** *Anal. Biochem.,* 1994, vol. 219, 87-95
- **EDDY, N.B. et al.** *J. Pharm. Exp. Therap.,* 1953, vol. 107, 385-389